(19)

Europäisches Patentamt
European Patent Office
Office européen des brevets

(11) EP 3 213 672 A1

(12) **EUROPEAN PATENT APPLICATION**
published in accordance with Art. 153(4) EPC

(43) Date of publication:
06.09.2017 Bulletin 2017/36

(51) Int Cl.:
*A61B 5/00* (2006.01)  *A61B 5/107* (2006.01)

(21) Application number: 15853990.8

(22) Date of filing: 09.10.2015

(86) International application number:
PCT/JP2015/078711

(87) International publication number:
WO 2016/067892 (06.05.2016 Gazette 2016/18)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 28.10.2014 JP 2014219467

(71) Applicant: Konica Minolta, Inc.
Tokyo 100-7015 (JP)

(72) Inventor: MATSUDA, Shinya
Tokyo 100-7015 (JP)

(74) Representative: Alton, Andrew
Urquhart-Dykes & Lord LLP
Arena Point
Merrion Way
Leeds LS2 8PA (GB)

(54) **DEGREE-OF-HEALTH OUTPUTTING DEVICE, DEGREE-OF-HEALTH OUTPUTTING SYSTEM, AND PROGRAM**

(57) A degree-of-health outputting device (1) is provided with a display unit (4), an operating unit (5), and an output unit. The display unit (4) displays a captured image of a region, the state of which changes according to a degree of health in a living body, and displays a plurality of comparison images having different degrees of health obtained on the basis of a reference image of the region captured prior to the time the abovementioned captured image was acquired for the same living body. The operating unit (5) is provided in order to select one of the plurality of comparison images displayed by the display unit (4). The output unit (e.g., the display unit (4)) outputs (e.g., displays) information relating to the degree of health of the living body on the basis of the comparison image selected through use of the operating unit (5).

FIG.19

## Description

### Technical Field

The present invention relates to a degree-of-health outputting device which outputs information as to the degree of health of a living subject, a degree-of-health outputting system that incorporates such a degree-of-health outputting device, and a program for making a computer function as a degree-of-health outputting device.

### Background Art

Diagnosis based on images of living subjects is widely practiced in Western medicine, and provides a basis not only for diagnosis of skin on the body surface, such as the face and limbs, but also for diagnosis of membranous tissues, such as the lips and eyelids, and for diagnosis of digestive and other internal organs, such as the stomach and intestines, using X-rays and ultrasonic waves. Such diagnosis is performed by doctors with adequate education. In addition to education based on literature and the like, clinical diagnosis of numerous patients leads to accumulated knowledge as to abnormal conditions, enabling accurate diagnosis.

Today, attempts to automatize such diagnosis through numerical analysis using a computer are studied, but are all far from reaching the level of experienced specialized doctors.

On the other hand, in Oriental medicine, diagnosing methods (visual diagnosis, tongue diagnosis) are known whereby health or disease condition is diagnosed through diagnosis of the condition of the face or tongue. For example, in tongue diagnosis, physical condition and the degree of health are diagnosed based on the color and shape of the tongue and of the tongue coating. That is, diagnosis items in tongue diagnosis include tongue color, tongue thickness, tongue coating color, and tongue coating thickness, and the like.

In healthy condition, the tongue has a pale pink color, the tongue coating has a pale white color, and the tongue and the tongue coating each have a standard thickness. Poor blood circulation and poor water metabolism, fever, and compromised immunity cause changes in those colors and shapes. These are diagnosed by specialized doctors, who, however, rely on experience and intuition; thus, the diagnosis varies from one individual to another and lacks in objectivity. To make diagnosis accurate and objective, there have conventionally been proposed devices and appliances for assisting diagnosis of patients.

For example, in the tongue diagnosis assisting device disclosed in Patent Document 1, tongue images (sample images) of a plurality of other people are classified into a plurality of groups according to attributes (for example, cold, heat, real, imaginary, and other attributes) and stored in a storage unit. Out of those groups, one that matches the attributes of a patient's tongue is identified, and out of that group, a tongue image that matches the patient's tongue is identified. Then, a method of treatment corresponding to the identified tongue image is outputted (for example, displayed). In this way, the diagnosis of the patient is assisted.

Patent Document 2 discloses a mirror appliance in which a plurality of specimens are arranged around a mirror. As the plurality of specimens, for example, stickers created based on photographs under the supervision of doctors and the like are used. An object (for example, the color of an examinee's tongue) seen in the mirror is compared with the plurality of specimens (for example, the colors of other people's tongues), and in this way, the examinee's health condition can be grasped easily.

Patent Document 3 discloses a system in which a tongue image of a patient and a standard tongue image are projected close together to make their comparison easy and to make accurate diagnosis possible. Patent Document 4 discloses a device that displays a diagnosis target image along with an image of a similar symptom and a diagnosis result to assist diagnosis.

### List of Citations

### Patent Literature

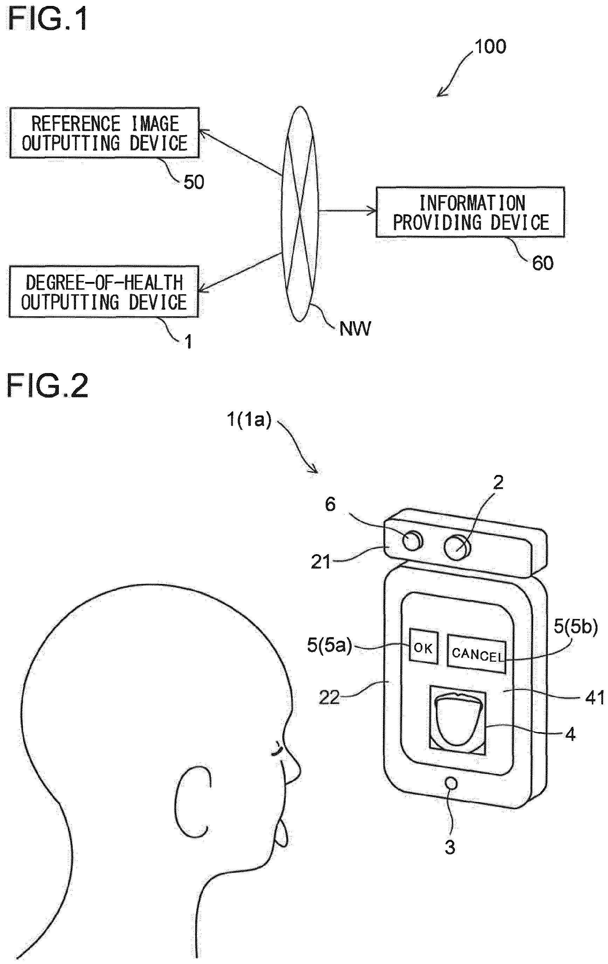

Patent Document 1: Japanese Patent Application published as No. 2013-17660 (see claims 1 and 7, paragraph 0023, etc.)
Patent Document 2: Japanese Patent registered as No. 5150440 (see claims 1 to 3, paragraphs 0013 and 0015 to 0021, Fig. 2, etc.)
Patent Document 3: Japanese Patent registered as No. 2763989 (see claim 1, paragraph 0018, Fig. 1, etc.)
Patent Document 4: Japanese Patent Application published as No. 2007-279942 (see claim 1, paragraph 0042, Fig. 4, etc.)

**Summary of the Invention**

**Technical Problem**

According to the prior art mentioned above, through comparison of a symptom of a patient (examinee as the target of diagnosis) with symptoms of other people, the examinee's health condition is diagnosed and grasped. This is advantageous in permitting an unexperienced or non-specialized doctor to make a certain diagnosis. However, according to the prior art mentioned above, since comparison is performed with other people's symptoms (images), it is difficult to distinguish whether a difference in health condition between the examinee and other people is due to the examinee's individual difference from them or due to a disease. Moreover, the degree and progress of a disease and the change in an image vary from one individual to another. Thus, it is difficult to grasp the examinee's degree of health (level of disease) accurately.

Patent Document 2 teaches that it is also possible to use, as the plurality of specimens stuck around the mirror, those created based on pictures of the examinee's own tongue shot with a camera. However, in that case, specimens can be created only up to the upper limit or down to the lower limit of the range of variation of the examinee's health condition in the past. Thus, if the examinee's health condition at the time of diagnosis falls outside the range of variation, it is impossible to grasp the degree of health.

The above-mentioned problems can occur likewise in the diagnosis of an animal other than a human.

Devised to solve the above-mentioned problems, the present invention aims to provide a degree-of-health outputting device that permits the user to grasp the degree of health of a living subject as a target of diagnosis accurately without being influenced by an individual difference from other living subjects (other people or other animals), a degree-of-health outputting system, and a program for making a computer function as a degree-of-health outputting device.

**Means for Solving the Problem**

According to one aspect of the present invention, a degree-of-health outputting device includes: a display unit configured to display together a shot image obtained by shooting a part whose condition changes according to the degree of health in a living subject and a plurality of comparison images with different degrees of health obtained based on a reference image obtained by shooting the part before the time of acquisition of the shot image with respect to the same living subject; an operation unit configured to be operated to select one of the plurality of comparison images displayed on the display unit; and an output unit configured to output information as to the degree of health of the living subject based on the comparison image selected on the operation unit.

According to another aspect of the present invention, a program for making a computer execute: a process of displaying, on a display unit, a shot image obtained by shooting a part whose condition changes according to the degree of health in a living subject and a plurality of comparison images with different degrees of health obtained based on a reference image obtained by shooting the part before the time of acquisition of the shot image with respect to the same living subject; and a process of, when one of the plurality of comparison images displayed on the display unit is selected on an operation unit, outputting, from an output unit, information as to the degree of health of the living subject based on the selected comparison image.

According to another aspect of the present invention, a degree-of-health outputting system includes: a degree-of-health outputting device as described above; and an information providing device. Here, the information providing device includes: a comparison image creation unit configured to create the plurality of comparison images from the reference image by image processing; and a communication unit configured to output data of the plurality of comparison images to the degree-of-health outputting device.

According to another aspect of the present invention, a degree-of-health outputting device includes: a display unit configured to display together a shot image obtained by shooting a part whose condition changes according to the degree of health in a living subject, a plurality of comparison images with different degrees of health obtained based on a reference image obtained by shooting the part before the time of acquisition of the shot image with respect to the same living subject, and information as to the degree of health of the living subject corresponding to each of the plurality of comparison images.

According to another aspect of the present invention, a degree-of-health outputting system includes: a degree-of-health outputting device as described above; and an information providing device. Here, the information providing device includes: a comparison image creation unit configured to create the plurality of comparison images from the reference image by image processing; a storage unit configured to store a table defining the correspondence between each of the plurality of comparison images and the information as to the degree of health; and a communication unit configured to output the data of the plurality of comparison images and the information as to the degree of health corresponding to each of the plurality of comparison images.

**Advantageous Effects of the Invention**

With a configuration as described above, it is possible to permit the user to grasp the degree of health of a living subject as a target of diagnosis accurately without being influenced by an individual difference from other living subjects.

**Brief Description of Drawings**

Fig. 1 is an explanatory diagram showing an outline configuration of a degree-of-health outputting system according to one embodiment of the present invention;

Fig. 2 is a perspective view showing an exterior appearance of an organ image shooting device as one example of a degree-of-health outputting device in the above degree-of-health outputting system;

Fig. 3 is a block diagram showing an outline configuration of the above organ image shooting device;

Fig. 4 is an explanatory diagram showing a positional relationship between an illumination unit and an imaging unit with respect to a shooting target;

Fig. 5 is an explanatory diagram showing one example of a shot image of a tongue and an edge extraction filter and a contour line of the tongue extracted with the filter;

Fig. 6 is a block diagram showing an outline configuration of a reference image outputting device in the above degree-of-health outputting system;

Fig. 7 is a block diagram showing an outline configuration of an information providing device in the above degree-of-health outputting system;

Fig. 8 is an explanatory diagram showing typical diagnosis items as to the tongue and the tongue coating;

Fig. 9 is an explanatory diagram showing one example of a plurality of comparison images created as to tongue color;

Fig. 10 is a graph showing a tongue color in an RG chromaticity coordinate system;

Fig. 11 is an explanatory diagram showing regions on a tongue in a reference image;

Fig. 12 is an explanatory diagram showing one example of a plurality of comparison images created as to tongue shape;

Fig. 13 is an explanatory diagram showing one example of a plurality of comparison images created as to tongue coating color;

Fig. 14 is a graph showing a tongue coating color in an RG chromaticity coordinate system;

Fig. 15 is an explanatory diagram showing one example of a plurality of comparison images created as to tongue coating thickness;

Fig. 16 is an explanatory diagram schematically showing reference images obtained by shooting a tongue under different illumination light sources;

Fig. 17 is an explanatory diagram schematically showing comparison images before and after correction;

Fig. 18 is a flow chart showing a flow of operation for creating comparison images in the above degree-of-health outputting system;

Fig. 19 is a flow chart showing a flow of operation for determining a degree of health in the above degree-of-health outputting system;

Fig. 20 is an explanatory diagram schematically showing a state where a shot image and a plurality of comparison images are displayed together on a display unit as to tongue coating color;

Fig. 21 is an explanatory diagram schematically showing a state where a shot image and a plurality of comparison images are displayed together on a display unit as to tongue shape;

Fig. 22 is a flow chart showing another flow of operation for determining a degree of health in the above degree-of-health outputting system;

Fig. 23 is an explanatory diagram showing an example of a display screen of questionnaire items;

Fig. 24 is an explanatory diagram schematically showing a state where a shot image, a plurality of comparison images, and information on the degrees of health corresponding to the comparison images are displayed in a display unit as to tongue coating color;

Fig. 25 is an explanatory diagram showing another configuration of the above degree-of-health outputting system;

Fig. 26 is a block diagram showing an outline configuration of an organ image shooting device as an degree-of-health outputting device in the above degree-of-health outputting system of the other configuration;

Fig. 27 is a flow chart showing a flow of operation for creating comparison images in the above degree-of-health outputting system;

Fig. 28 is a flow chart showing a flow of operation for determining a degree of health in the above degree-of-health outputting system;

Fig. 29 is an explanatory diagram schematically showing an image obtained by observing the inner lining of a large intestine with an endoscope; and

Fig. 30 is an explanatory diagram showing one example of a plurality of comparison images created based on the above image.

**Description of Embodiments**

An embodiment of the present invention will be described below with reference to the accompanying drawings. In the present description, it is assumed that a numerical range from A to B is inclusive of the lower limit value A and the upper limit value B.

[Outline Configuration of a System]

Fig. 1 is an explanatory diagram showing an outline configuration of a degree-of-health outputting system 100 according to the embodiment. The degree-of-health outputting system 100 is composed of a degree-of-health outputting device 1, a reference image outputting device 50, and an information providing device 60 that are connected together across a communication network NW. As the communication network NW, for example, the Internet can be used. The communication network NW may be wired or wireless.

The degree-of-health outputting device 1 is a device that displays a plurality of comparison images with different degrees of health and that outputs, based on a comparison image selected by the user, the degree of health (level of disease) of a living subject which is the target of diagnosis. The data of the plurality of comparison images is created, for example, in the information providing device 60, and is then fed to the degree-of-health outputting device 1. Here, the user who operates the degree-of-health outputting device 1 may be the doctor who diagnoses the living subject, or the living subject himself (examinee) that is the target of diagnosis.

The degree-of-health outputting device 1 can be configured, for example, as a multifunction portable terminal (computer), such as a smartphone, on which a predetermined program (application software) has been installed. That is, by letting the computer read and execute the program, it is possible to make the degree-of-health outputting device 1 perform various kinds of processing as will be described later. Such as program can be acquired, for example, by being downloaded across the network.

The reference image outputting device 50 is configured, for example, as a personal computer. The reference image outputting device 50 acquires a reference image, based on which the plurality of comparison images are produced, and then feeds the data of the reference image to the information providing device 60. A reference image is an image of the same living subject as the target of diagnosis as acquired by shooting it in healthy condition in the past. Here, the "past" denotes a time point earlier than a reference time point (present) which is the time of acquisition (shooting) of a shot image obtained by shooting, with the degree-of-health outputting device 1, a part (for example, the tongue) whose condition changes according to the living subject's degree of health.

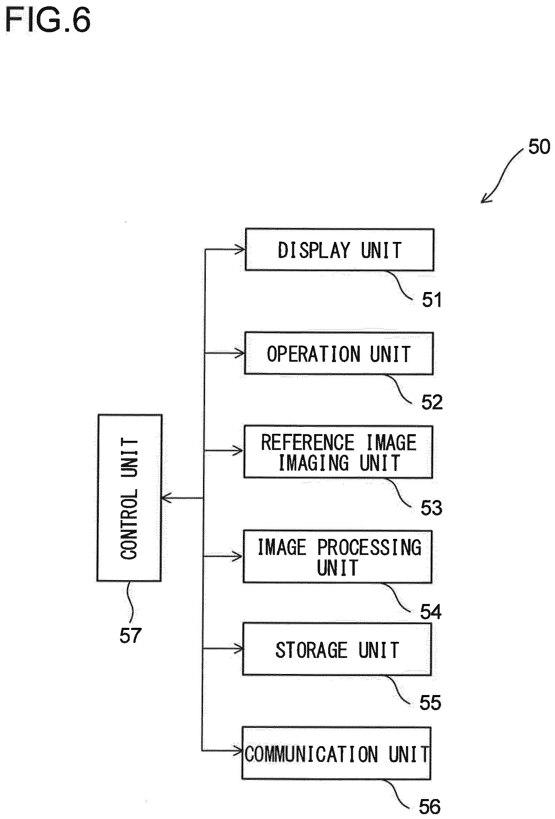

The reference image may be, for example, an image acquired by shooting with a digital camera and fed to the reference image outputting device 50 via a USB cable or a recording medium, or an image acquired by shooting with a web camera (see the reference image imaging unit 53 (see Fig. 6) described later) fitted optionally to, or built in, the reference image outputting device 50, or an image transmitted, attached to e-mail, from another device (for example, another personal computer) to the reference image outputting device 50.

The information providing device 60 is a server (computer) that creates, by image processing, the plurality of comparison images from the data of the reference image fed from the reference image outputting device 50 and that then provides (feeds) the resulting data to the degree-of-health outputting device 1.

Now, the degree-of-health outputting device 1, the reference image outputting device 50, and the information providing device 60 will be described in detail one by one.

[Overall Configuration of the Degree-of-Health Outputting Device]

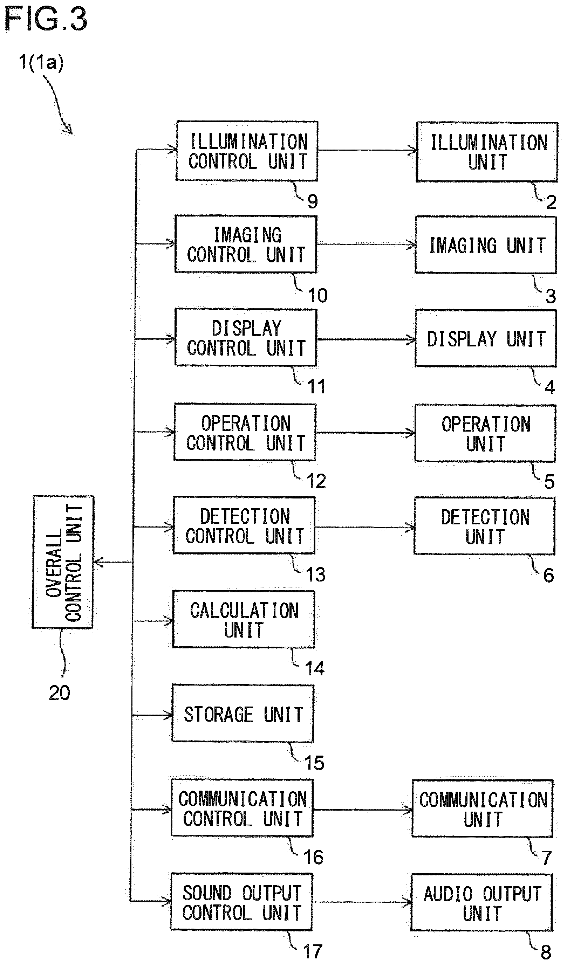

Fig. 2 is a perspective view showing the exterior appearance of an organ image shooting device 1a as one example of the degree-of-health outputting device 1 according to the embodiment. Fig. 3 is a block diagram showing an outline configuration of the organ image shooting device 1a. The organ image shooting device 1a includes an illumination unit 2, an imaging unit 3, a display unit 4, an operation unit 5, a detection unit 6, a communication unit 7, and an audio output unit 8. The illumination unit 2 and the detection unit 6 are provided in a housing 21, and the imaging unit 3, the display unit 4, the operation unit 5, the communication unit 7, and the audio output unit 8 are provided in a housing 22. The housings 21 and 22 are coupled together so as to be rotatable relative to each other, though the rotating capability is not essential here; instead, one may be completely fixed to the other. The illumination unit 2 and the like enumerated above may be provided in a single housing.

The illumination unit 2 is for illuminating an organ (here, the tongue) of a living subject as a shooting target, and is configured as an illuminator that illuminates the shooting target from above. Used as the light source for the illumination unit 2 is, for example, one that emits daylight color light, such as a xenon lamp. The brightness of the light source, though it depends on the sensitivity of the imaging unit 3 and the distance to the shooting target, can be set, for example, such that the illuminance on the shooting target is 1000 to 10000 lx. The illumination unit 2 includes, in addition to the light source, a lighting circuit and a dimming circuit so that its lighting/extinguishing and dimming can be controlled according to instructions from an illumination control unit 9.

The imaging unit 3 is for acquiring an image (shot image) by shooting, under the illumination by the illumination unit 2, a part (for example, the tongue) whose condition changes according to the degree of health of a subject person (examinee) whose degree of health is to be determined. The imaging unit 3 includes an imaging lens and an area sensor (image sensor). The imaging lens has its aperture (lens brightness), shutter speed, and focal length so set that the entire range of the shooting target is in focus. For example, the f-number is set at 16, the shutter speed at 1/120 seconds, and the focal length at 20 mm.

The area sensor comprises, for example, an image sensor such as a CCD (charge-coupled device) image sensor or a CMOS (complementary metal-oxide-semiconductor) image sensor, and its sensitivity and resolution are so set as to enable sufficient detection of the color and shape of the shooting target. For example, the sensitivity is set at 60 dB, and the resolution at ten megapixels.

The shooting by the imaging unit 3 is controlled by an imaging control unit 10. The imaging unit 3 includes, in addition to the imaging lens and the area sensor, a focusing mechanism, an aperture mechanism, a driving circuit, an A/D converter, and the like, of which none are illustrated, so that its focus and aperture and the A/D conversion it performs are controlled according to instructions from the imaging control unit 10. The imaging unit 3 acquires, as the data of a shot image, for example, eight-bit data ranging from 0 to 255 for each of red (R), green (G), and blue (B). That is, the imaging unit 3 acquires, for each of different colors (RGB), an image obtained by shooting the shooting target, and thereby acquires image data of the different colors.

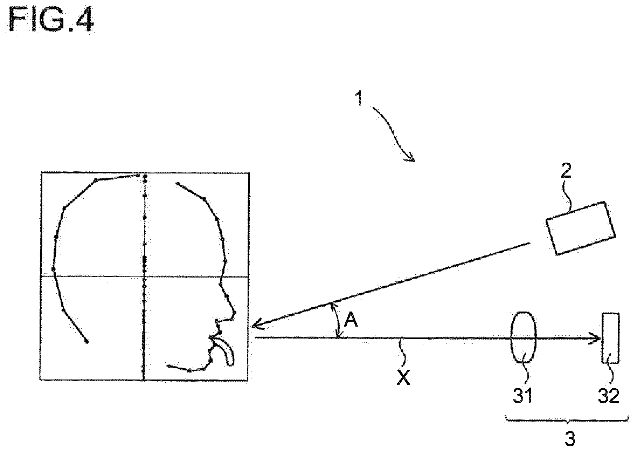

Fig. 4 is an explanatory diagram showing the positional relationship of the illumination unit 2 and the imaging unit 3 relative to the shooting target (the tongue or the face). As shown there, the imaging unit 3, which includes at least an imaging lens 31 and an area sensor 32, is arranged right in front of the shooting target. The illumination unit 2 is arranged so as to illuminate the shooting target at an angle A of, for example, 0° to 45° relative to the shooting optical axis X of the imaging unit 3 which passes through the shooting target. The shooting optical axis X denotes the optical axis of the imaging lens 31 provided in the imaging unit 3.

A large angle A during illumination leads to higher measurement accuracy of surface irregularities but to a smaller shooting range of the tongue due to the shadow of the upper lip. In contrast, a small angle A leads to a larger shooting range but to lower measurement accuracy and also to severe white clipping owing to regular reflection of the illumination light. Out of these consideration, a preferred range of the angle A during illumination is from 15° to 30°.

The display unit 4 includes a liquid crystal panel, a backlight, a lighting circuit, and a control circuit, of which none are illustrated, and displays, for each of different degree-of-health diagnosis items, the shot image acquired by shooting with the imaging unit 3 along with a plurality of comparative images with different degrees of health that are created in the information providing device 60. The diagnosis items include at least one of tongue color, tongue shape, tongue coating color, and tongue coating thickness.

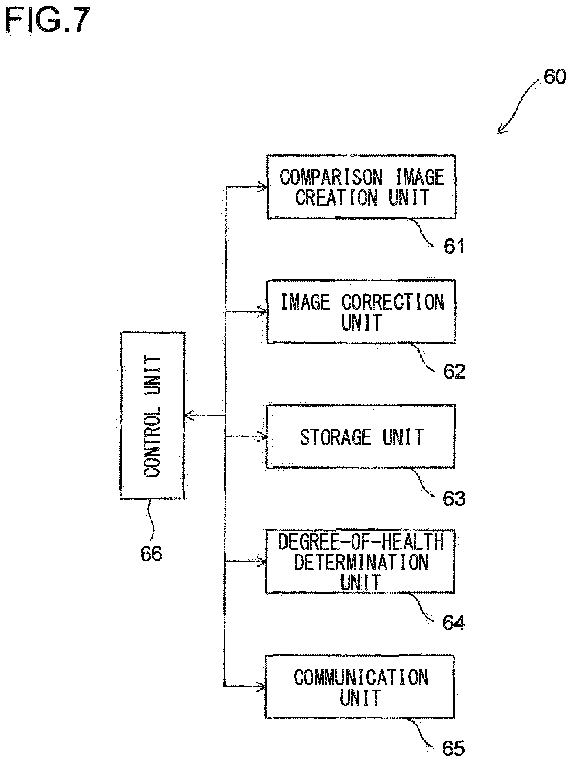

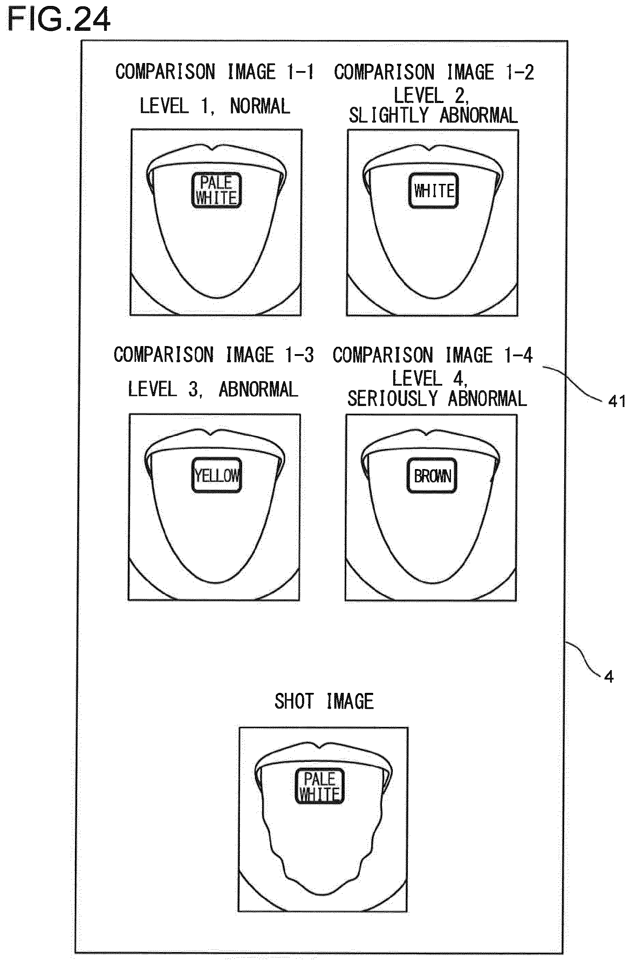

The display unit 4 also functions as an output unit that, when one of the plurality of comparison images displayed on it is selected on the operation unit 5, outputs information as to the degree of health of the living subject based on the selected comparison image. A table defining the correspondence between different comparison images and different degrees of health is stored in a storage unit 63 (see Fig. 7) in the information providing device 60, which create a plurality of such comparison images. Accordingly, transmitting information (number) on the comparison image selected on the operation unit 5 to the information providing device 60 and making the information providing device 60 transmit information as to the degree of health corresponding to the selected comparison image to the degree-of-health outputting device 1 enables the display unit 4 to output (display) information as to the transmitted degree of health (for example, normal, poor blood circulation, abnormal water metabolism, and the like). The display of various kinds of information on the display unit 4 is controlled by a display control unit 11. The display unit 4 may display together the plurality of comparison images along with information as to the corresponding degrees of health of the living subject (level 1 for normal, level 2 for slightly abnormal, level 3 for abnormal, and level 4 for seriously abnormal) (see Fig. 24).

The operation unit 5 is an input unit for requesting shooting by the imaging unit 3, and is composed of an OK button (shooting execution button) 5a and a CANCEL button 5b. In the embodiment, the display unit 4 and the operation unit 5 are configured as a touch-panel display device 41 (see Fig. 2) common to them. The display on the operation unit 5 in the touch-panel display device 41 is controlled by an operation control unit 12. The operation unit 5 may be configured as any input unit other than the touch-panel display device 41 (the operation unit 5 may be provided outside the display region of the touch-panel display device 41).

The operation unit 5 functions also as a selection unit for allowing the user to select one of the plurality of comparison images displayed on the display unit 4. That is, when a plurality of comparison images are displayed on the display unit 4, the user can, by pressing a position on one of the comparison images on the operation unit 5 (the touch-panel display device 41), select the comparison image at the pressed position.

The detection unit 6 is for detecting the color temperature of the light in the ambience around the above-mentioned part (for example, the tongue) of the shooting target illuminated by the illumination unit 2. The detection unit 6 is composed of RGB color sensors with a white semitransparent cover arranged on their light entrance side, and detects (measures) the color temperature of the ambient light based on the balance of the outputs from those sensors reflecting the amounts of light they respectively receive. The detection of the color temperature in the detection unit 6 is controlled by a detection control unit 13.

Information on the color temperature detected in the detection unit 6 is transmitted via the communication unit 7 to the information providing device 60. Thus, the information providing device 60 can, based on the color temperature at the time of acquisition of the shot image thus transmitted and the color temperature at the time of acquisition of the reference image used to create the plurality of comparison images, grasp the chromaticity (saturation and hue) of the shot image and of the plurality of comparison images, and can then correct, for example, the plurality of comparison images such that their chromaticity is closer together.

The communication unit 7 is an interface for transmitting information obtained in the degree-of-health outputting device 1 to the information providing device 60 across the communication network NW and for receiving information outputted from the information providing device 60. The transmission and reception of information in the communication unit 7 are controlled by a communication control unit 16. Information obtained in the degree-of-health outputting device 1 includes, as described above, information obtained through selection or input on the operation unit 5, the image data of the shot image acquired in the imaging unit 3, information as to the color temperature at the time of acquisition of the shot image detected in the detection unit 6, and the like. Information outputted from the information providing device 60 includes the data of the plurality of comparison images and information as to the degree of health and a remedy corresponding to it.

The audio output unit 8 is for audibly outputting various kinds of information, and comprises, for example, a loudspeaker. In particular, the audio output unit 8 can audibly output information on the degree of health and the like transmitted from the information providing device 60. In this case, it can be said that, like the display unit 4, the audio output unit 8 functions as an output unit that outputs information as to the degree of health of the living subject based on the comparison image selected on the operation unit 5. The audio output in the audio output unit 8 is controlled by an audio output control unit 17.

The organ image shooting device 1a also includes the illumination control unit 9, the imaging control unit 10, the display control unit 11, the operation control unit 12, the detection control unit 13, a calculation unit 14, a storage unit 15, the communication control unit 16, the audio output control unit 17, and an overall control unit 20 which controls all those different parts. As mentioned above, the illumination control unit 9, the imaging control unit 10, the display control unit 11, the operation control unit 12, the detection control unit 13, the communication control unit 16, and the audio output control unit 17 control the illumination unit 2, the imaging unit 3, the display unit 4, the operation unit 5, the detection unit 6, the communication unit 7, and the audio output unit 8 respectively. The overall control unit 20 comprises, for example, a CPU (central processing unit), and executes operation programs stored in the storage unit 15. The illumination control unit 9, the imaging control unit 10, the display control unit 11, the operation control unit 12, the detection control unit 13, the communication control unit 16, the audio output control unit 17, and the overall control unit 20 may be configured integrally (for example, as a single CPU).

The calculation unit 14 is a processing circuit which performs processing for extracting the contour line of an organ from an image acquired in the imaging unit 3. In the embodiment, the calculation unit 14 extracts the contour line of the tongue as an organ based on luminance edges (parts where luminance sharply changes) in the shot image.

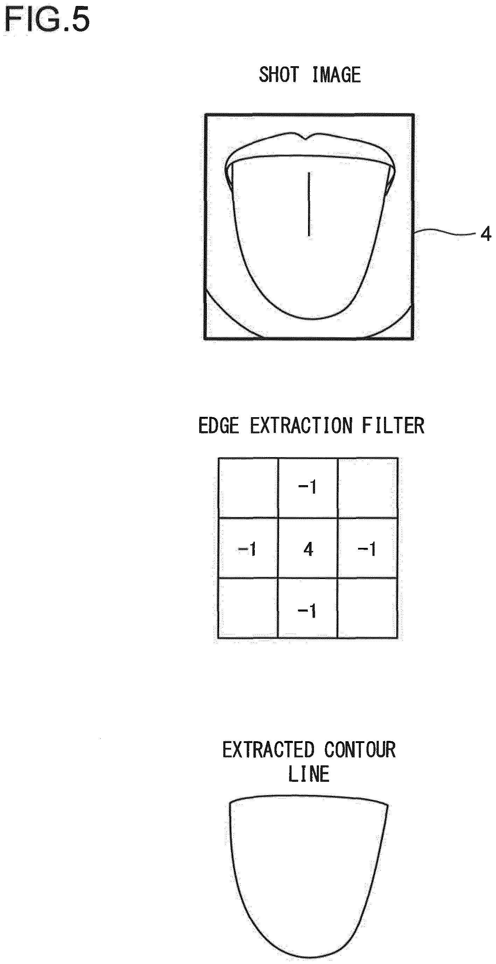

Extraction of a luminance edge can be achieved, for example, by use of an edge extraction filter as shown in Fig. 5. An edge extraction filter is a filter that gives weights to pixels around a pixel of interest while performing first derivation (while taking differences in image data between adjacent pixels). By use of such an edge extraction filter, for example, with respect to the G image data of the individual pixels of the shot image, by taking differences in image data between the pixel of interest and pixels around it and then extracting those pixels for which the differences exceed a predetermined threshold value, it is possible to extract pixels that form luminance edges. Around the tongue, its shadow produces luminance differences; thus, by extracting pixels forming luminance edges as described above, it is possible to extract the contour line of the tongue. Although, here, G image data, which has a largest influence on luminance, is used in calculation, R or B image data may be used instead.

The storage unit 15 is a memory that stores the data of the image acquired in the imaging unit 3, data acquired in the calculation unit 14, information received from the information providing device 60, and the like and that also stores programs for making the above-mentioned different control units operate. Such a memory can be configured to include RAM, ROM, nonvolatile memory, and the like.

[Configuration of the Reference Image Outputting Device]

Next, the reference image outputting device 50 will be described in detail. Fig. 6 is a block diagram showing an outline configuration of the reference image outputting device 50. The reference image outputting device 50 includes a display unit 51, an operation unit 52, a reference image imaging unit 53, an image processing unit 54, a storage unit 55, a communication unit 56, and a control unit 57. The control unit 57 comprises, for example, a CPU, and controls the operation of different parts in the reference image outputting device 50.

The display unit 51 is a display (for example, an LCD) that displays various kinds of information including the image shot in the reference image imaging unit 53. The operation unit 52 includes a keyboard and a mouse, and is provided to allow entry of a request for shooting by the reference image imaging unit 53 and a request for transmission of the data of the shot image to the information providing device 60 and to allow input of various kinds of information.

The reference image imaging unit 53 shoots, with respect to the same living subject as that shot by the degree-of-health outputting device 1, a part whose condition varies according to the degree of health, and acquires the above-mentioned reference image based on which the plurality of comparison images are created in the information providing device 60. The reference image imaging unit 53 comprises, for example, a built-in web camera. The reference image imaging unit 53 is not an essential component; instead, an image shot with an external digital camera may be used as a reference image, and its data may be fed into the reference image outputting device 50 via a recording medium or a USB cable.

The image processing unit 54 is a processing circuit which cuts out a necessary region from the reference image acquired by shooting in the reference image imaging unit 53. For example, when the entire face of a living subject is shot in the reference image imaging unit 53, from the image of the entire face, the image of a region including a part (for example, the tongue) whose condition varies according to the degree of health is cut out. This processing is achieved, for example, by recognizing the region (for example, red) of the tongue in the RGB image data of the shot image and cutting out from it the image of a region that includes the tongue and that is smaller in size than the entire face. In the following description, the image thus cut out is referred to as the reference image.

The storage unit 55 is a memory that stores the data of the reference image acquired in the reference image imaging unit 53, programs for making the different parts of the reference image outputting device 50 operate, and the like. Such a storage unit 55 can be configured to include RAM, ROM, nonvolatile memory, and the like. In a case where the data of the reference image is acquired via a recording medium or the like, or where it is received from another device in the form of a file attached to e-mail, such data also is stored in the storage unit 55. Moreover, in a case where information on the color temperature of the ambient light at the time of acquisition of the reference image is received from another device, that information also is stored in the storage unit 55. Here, the information on the color temperature may be the values (detected RGB values) as they are detected by an external sensor at the time of acquisition of the reference image, or may be the RGB data of an image obtained by shooting white paper.

The communication unit 56 is an interface for receiving, from a device other than the degree-of-health outputting device 1 and the information providing device 60, the data of the reference image and the above-mentioned information on the color temperature, and for transmitting to the information providing device 60 the data of the reference image and the information on the color temperature of the ambient light at the time of acquisition of the reference image that are stored in the storage unit 55.

[Configuration of the Information Providing Device]

Next, the information providing device 60 will be described in detail. Fig. 7 is a block diagram showing an outline configuration of the information providing device 60. The information providing device 60 includes a comparison image creation unit 61, an image correction unit 62, a storage unit 63, a degree-of-health determination unit 64, a communication unit 65, and a control unit 66. The control unit 66 comprises, for example, a CPU, and controls the operation of different parts in the information providing device 60.

The comparison image creation unit 61 is a processing circuit which creates, by image processing, a plurality of comparison images with different degrees of health from the data of the reference image transmitted from the reference image outputting device 50 and received via the communication unit 65. The method for creating the plurality of comparison images will be described later.

Based on the color temperature at the time of acquisition of the shot image transmitted from the degree-of-health outputting device 1 (organ image shooting device 1a) and the color temperature at the time of acquisition of the reference image transmitted from the reference image outputting device 50, the image correction unit 62 is a processing circuit which corrects the plurality of comparison images so as to bring the overall color of the comparison images closer to the color of an image shot under the same illumination as at the time of acquisition of the shot image. The method for correcting the comparison images will be described later.

The storage unit 63 is a memory that stores the data of the reference image transmitted from the reference image outputting device 50, the data of the plurality of comparison images created in the comparison image creation unit 61, the data of the plurality of comparison images corrected in the image correction unit 62, information (such as information on the selected comparison image and information on the color temperature at the time of acquisition of the shot image) transmitted from the degree-of-health outputting device 1, and the like and that also stores programs for making the above-mentioned different parts operate. Such a memory can be configured to include RAM, ROM, nonvolatile memory, and the like. In particular, the data of the plurality of comparison images with different degrees of health is stored in the storage unit 63 in a form associated with the degrees of health of those comparison images respectively.

Based on the comparison image selected on the operation unit 5 in the degree-of-health outputting device 1, the degree-of-health determination unit 64 is a processing circuit which determines and outputs the degree of health of the living subject. That is, when one of the plurality of comparison images is selected on the operation unit 5 in the degree-of-health outputting device 1, selection information is transmitted to the information providing device 60. Thus, the degree-of-health determination unit 64 can, by reading from the storage unit 63 the degree of health corresponding to the selected comparison image, determine the degree of health.

[Method for Creating Comparison Images]

Next, the method for creating a plurality of comparison images will be described. The above-mentioned plurality of comparison images are images (grade specimens) obtained by varying, by image processing, the reference image over a plurality of grades with different degrees of health with respect to at least one diagnosis item that serves as an index in determining the degree of health of a living subject.

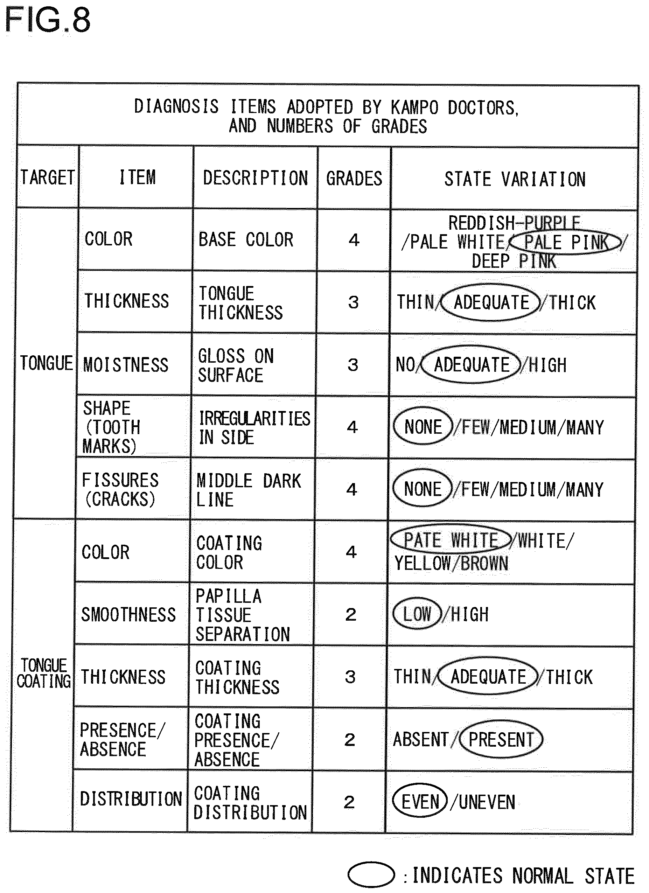

Fig. 8 shows typical diagnosis items as to the tongue and the tongue coating in Oriental medicine. In general, for the tongue, five aspects - the color of the tongue, the thickness of the tongue, moistness (gloss) on the tongue surface, the shape of the tongue (tooth marks), and fissures (cracks on the tongue surface) - are taken as diagnosis items. For the tongue coating, five aspects - the color of the tongue coating, the smoothness of the tongue coating (the degree of separation of papilla tissues), the thickness of the tongue coating, the presence/absence of the tongue coating, and the distribution of the tongue coating. Under "State Variation" in Fig. 8, a circle indicates the normal (healthy) state, any state away from it being abnormal (diseased).

Of the diagnosis items shown in Fig. 8, the following four are particularly important: tongue color, tongue shape (tooth marks), tongue coating color, and tongue coating thickness. Now, the method for creating a plurality of comparison images will be described for each of those diagnosis items.

(Comparative Images as to Tongue Color)

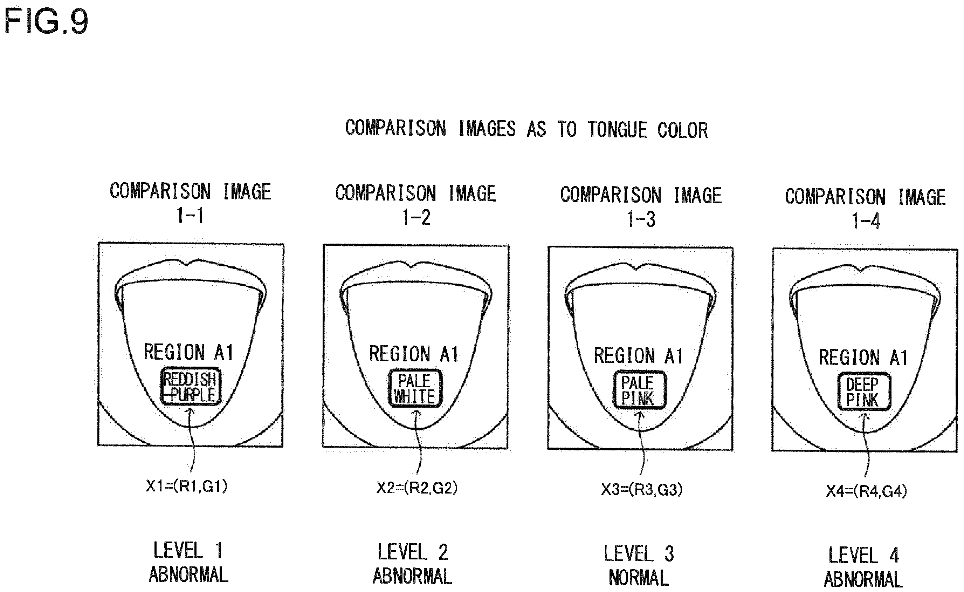

Fig. 9 shows an example of a plurality of comparison images created as to tongue color based on an image (reference image) taken when an examinee is healthy, showing cases where the tongue color is, from left to right, reddish-purple (level 1; abnormal), pale white (level 2, abnormal); pale pink (level 3; normal), and deep pink (level 4; abnormal). The tongue color, as observed in a lower middle part of the tongue, in a region A1 at or close to the tongue tip, varies between reddish-purple and deep pink. Accordingly, in a chromaticity coordinate system with R and G as two variables, the tongue color varies largely according to the formula below.

$$G = -0.5R + C \tag{1}$$

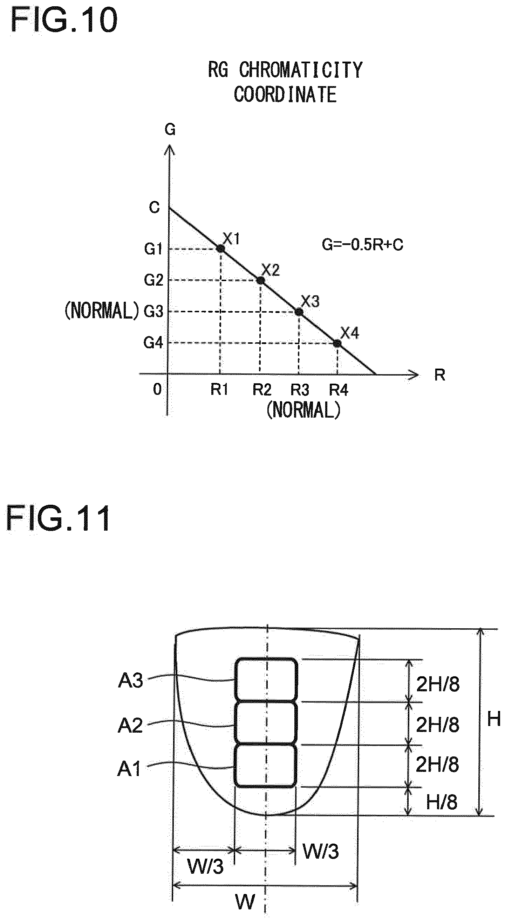

Here, R represents the proportion of R image data in the sum of RGB image data in region A1 on the tongue, that is, $(R / (R+G+B))$, G represents the proportion of G image data in the sum of RGB image data in region A1 on the tongue,, that is, $(G / (R+G+B))$, and C represents a constant. Fig. 10 is a graph showing formula (1) in the RG chromaticity coordinate.

The reference image is an image shot when the examinee is healthy, and the R and G proportions in region A1 at that time are calculated from the image data of region A1. Accordingly, by substituting the so calculated R and G proportions in formula (1), it is possible to calculate the value of the constant C, which is unique to the examinee, and thereby to make formula (1) definite.

Thus, by varying the color of region A1 on the tongue in the reference image among the colors at points X1 to X4 on the graph of formula (1) (the R and G combinations at those points being X1 = (R1, G1), X2 = (R2, G2), X3 = (R3, G3), and X4 = (R4, G4) respectively), it is possible to create four comparison images 1-1 to 1-4 with different degrees of health. Here, it is assumed that points X1 to X4 include the point (in the above example, point X3) that corresponds to the tongue color in healthy condition.

Fig. 11 shows regions A1 to A3 on the tongue in the reference image. As shown there, when a strip-form region extending in the up/down direction in a middle part of the tongue in the left/right direction is divided into three regions referred to by A1 to A3 from bottom, these regions A1 to A3 are defined to have dimensions, for example, as shown in Fig. 11 in terms of the left/right-direction width W and the up/down-direction length H of the region surrounded by the contour line of the tongue. Thus, the comparison image creation unit 61 in the information providing device 60 can, by a method similar to that by which the calculation unit 14 in the degree-of-health outputting device 1 extracts the contour line, extract the contour line of the tongue from the reference image and then, based on the width W and the length H of the extracted contour line, set regions A1 to A3.

In tongue diagnosis practiced in *Kampo* (the Japanese adaptation of traditional Chinese medicine), the tongue color is generally inspected in left and right end parts of the tongue that have no tongue coating or in a lower middle part of the tongue (near the tongue tip). However, left and right end parts of the tongue tend to be illuminated differently due to surface irregularities and thus with varying brightness, causing deviations of image data from those values that show the natural tongue color. With this taken into consideration, in the embodiment, the plurality of comparison images are created by varying the tongue color in a lower middle part of the tongue, in region A1.

(Comparative Images as to Tongue Shape)

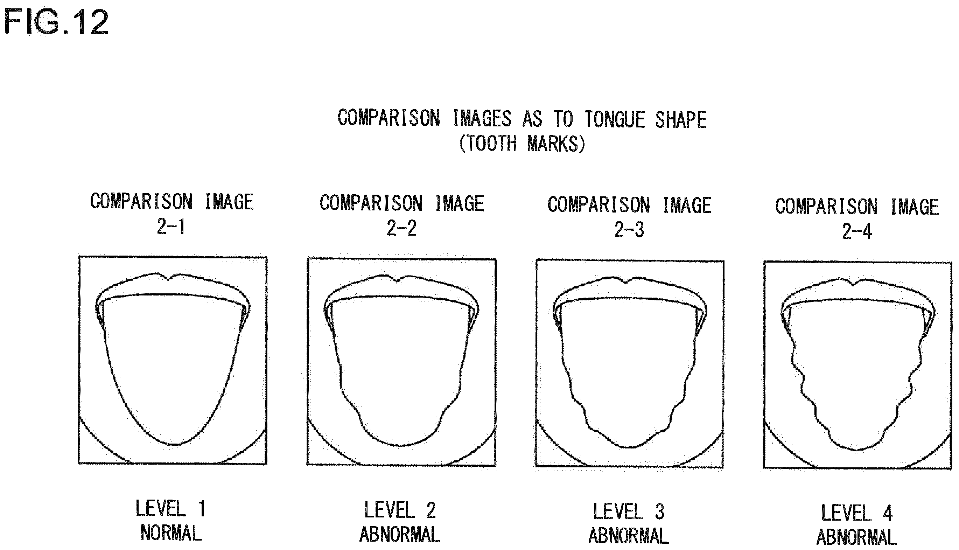

Fig. 12 shows an example of a plurality of comparison images created as to tongue shape (tooth marks) based on a reference image taken when an examinee is healthy, showing cases where the tongue has, from left to right, no tooth marks (level 1; normal), a few tooth marks (level 2; abnormal), more tooth marks (level 3; abnormal), and many tooth marks (level 4, abnormal).

A change in the tongue shape ascribable to tooth marks is produced as a result of the side of the tongue pressed against the inside of teeth. Thus, in the side of the tongue, irregularities (bumps and dents) with a pitch of several millimeters are formed as tooth marks. Accordingly, the comparison image creation unit 61 creates a plurality of comparison images by varying the depth of irregularities by moving, with a computer graphic technique, the contour line of the tongue detected as described above in the left/right direction. Considering that the unevenness due to tooth marks, that is, the depth of irregularities in the side of the tongue is about 2 mm at most, the comparison images 2-1 to 2-4 having tooth marks varied among four grades, namely levels 1 to 4, as described above can be created by varying the depth of irregularities among 0 mm, 1.2 mm, 1.6 mm, and 2 mm.

(Comparative Images as to Tongue Coating Color)

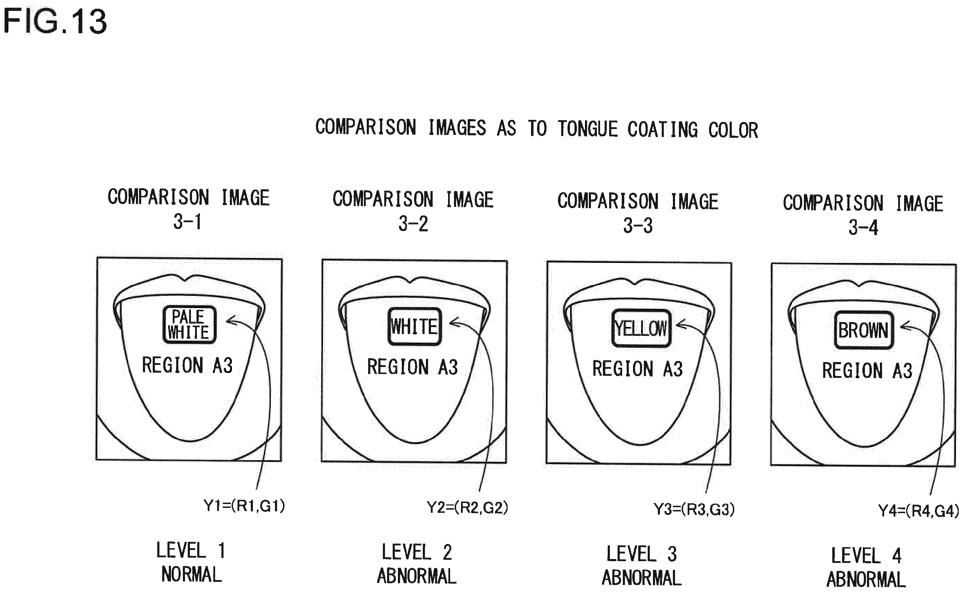

Fig. 13 shows an example of a plurality of comparison images created as to tongue coating color based on an image (reference image) taken when an examinee is healthy, showing cases where the tongue coating color is, from left to right, pale white (level 1; normal), white (level 2, abnormal); yellow (level 3; abnormal), and brown (level 4; abnormal). The tongue coating color, as observed in an upper middle part of the tongue, in a region A3 at or close to the tongue base, varies between pale white and brown. Accordingly, in a chromaticity coordinate with R and G as two variables, the tongue coating color varies largely according to the formula below.

$$G = C'R \qquad\qquad (2)$$

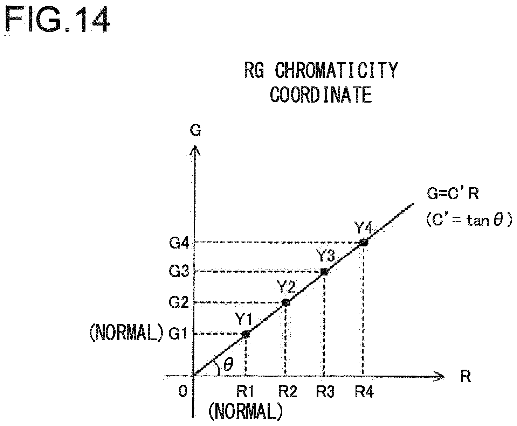

Here, R represents the proportion of R image data in the sum of RGB image data in region A3 on the tongue, that is, (R / (R+G+B)), G represents the proportion of G image data in the sum of RGB image data in region A3 on the tongue, that is, (G / (R+G+B)), and C' represents a constant. Fig. 14 is a graph showing formula (2) in the RG chromaticity coordinate.

The reference image is an image shot when the examinee is healthy, and the R and G proportions in region A3 at that time are calculated from the image data of region A3. Accordingly, by substituting the so calculated R and G proportions in formula (2), it is possible to calculate the value of the constant C', which is unique to the examinee, and thereby to make formula (2) definite.

Thus, by varying the color of region A3 on the tongue in the reference image among the colors at points Y1 to Y4 on the graph of formula (2) (the R and G combinations at those points being Y1 = (R1, G1), Y2 = (R2, G2), Y3 = (R3, G3), Y4 = (R4, G4)) respectively), it is possible to create four comparison images 3-1 to 3-4 with different degrees of health. Here, it is assumed that points Y1 to Y4 include the point (in the above example, point Y1) that corresponds to the tongue coating color in healthy condition.

The tongue coating forms through keratinization of papilla tissues in the mucous membrane on the tongue and it forms over an upper to a middle part of the tongue, in particular in an upper part. With this taken into consideration, in the embodiment, the plurality of comparison images are created by varying the tongue coating color in an upper middle part of the tongue, in region A3.

(Comparative Images as to Tongue Coating Thickness)

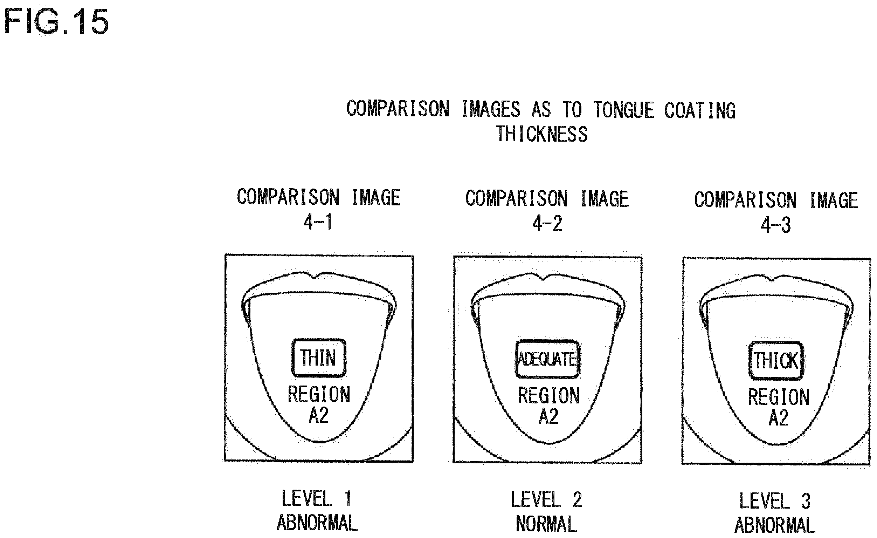

Fig. 15 is an example of a plurality of comparison images created as to tongue coating thickness based on an image (reference image) taken when an examinee is healthy, showing cases where the tongue coating is, from left to right, thin (level 1; abnormal), adequate (level 2; normal), and thick (level 3; abnormal). In Fig. 15, considering the difficulty illustrating the thickness of the tongue coating, different thicknesses of the tongue coating are labeled "thin", "adequate", and "thick".

A change in the tongue coating thickness can be determined by a change in the color of region A2 in a middle part of the tongue. That is, a large part of the tongue coating forms in region A3 in an upper middle part of the tongue as mentioned above, and thus the closer the color in region A2 in a middle part to the color in region A3 in an upper part, the thicker it is possible to determine that the tongue coating is. Accordingly, as to a change in the tongue coating color, as with a change in the tongue color expressed by formula (1), a larger R proportion indicates a smaller tongue coating thickness, and a larger G proportion indicates a larger tongue coating thickness.

Thus, by determining the R and G proportions in region A2 from the image data of region A2 on the tongue in the reference image shot when the examinee is healthy and substituting those proportions in formula (1), it is possible to calculate the value of the constant C, which is unique to the examinee, and thereby to make formula (1) definite. Then by varying the color in region A2 on the tongue in the reference image among the colors at three points on the graph of formula (1), it is possible to create the three comparison images 4-1 to 4-3 with different degrees of health. Here, it is assumed that those three points include the point (the point corresponding to the comparison image 4-2) that corresponds to the color of tongue coating in healthy condition.

[Correction Based on Color Temperature]

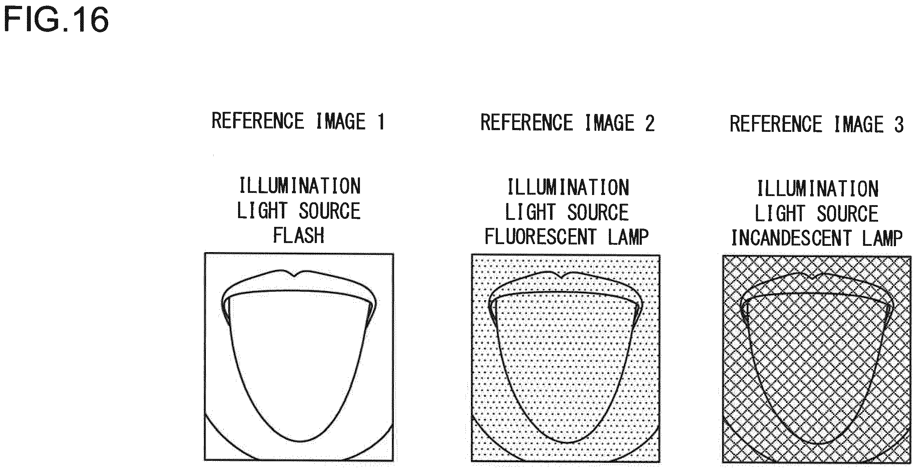

Next, correction of comparison images based on color temperature will be described. Fig. 16 schematically shows reference images obtained by shooting the tongue under, as an illumination light source, a camera flash, a fluorescent lamp, and an incandescent lamp respectively. When a reference image is acquired, as the illumination light differs, the chromaticity (saturation and hue) of the entire image of the reference image differs according to the illumination light. Thus, also the chromaticity of the entire image of the plurality of comparison images created from the reference image differs according to the illumination light at the time of acquisition of the reference image. Likewise, when the illumination light differs between at the time of acquisition (shooting) of a shot image in the degree-of-health outputting device 1 and at the time of acquisition (shooting) of a reference image, the tone color of the entire image differs between the shot image and the plurality of comparison images.

To cope with that, in the embodiment, when the color temperature of the illumination light (ambient light) at the time of acquisition of a shot image differs from the color temperature of the illumination light (ambient light) at the time of acquisition of a reference image, the image correction unit 62 in the information providing device 60 corrects at least either the shot image or a plurality of comparison images such that the chromaticity of the entire image of each of the created comparison images is closer, in relative terms, to the chromaticity of the entire image of the shot image. Here, the image correction unit 62 can grasp the color temperature at the time of acquisition of the shot image based on information transmitted from the degree-of-health outputting device 1, and can grasp the color temperature at the time of acquisition of the reference image based on information transmitted from the reference image outputting device 50.

One example of the method for such correction will now be described specifically. When there is a difference between the color temperature of the illumination light at the time of acquisition of a shot image and the color temperature of the illumination light at the time of acquisition of a reference image, the image correction unit 62 calculates the average value Rave of the R image data, the average value Gave of the G image data, and the average value Bave of the B image data of the entire image of the shot image, and also calculates, for each of the created comparison images, the average value R'ave of the R image data, the average value G'ave of the G image data, and the average value B'ave of the B image data of the entire image of the comparison image. Then the image correction unit 62 calculates, for each of the comparison images, an R correction coefficient (Rave/R'ave), a G correction coefficient (Gave/G'ave), and a B correction coefficient (Bave/B'ave), and multiplies the RGB image data of each pixel of the entire image of each comparison image by those correction coefficients respectively, thereby to correct each comparison image.

Correction similar to that described above may be performed on the shot image, or may be performed on both the shot image and the plurality of comparison images. In the former case, the denominator and the numerator of the correction coefficients are inverted compared with in the above-described example where the comparison images are corrected. In the latter case, it is possible, for example, by raising the image data (pixel values) of each pixel of the shot image through multiplication by correction coefficients while lowering the image data (pixel values) of each pixel of the comparison images through multiplication by correction coefficients, to bring closer together the average values of the RGB image data between the two.

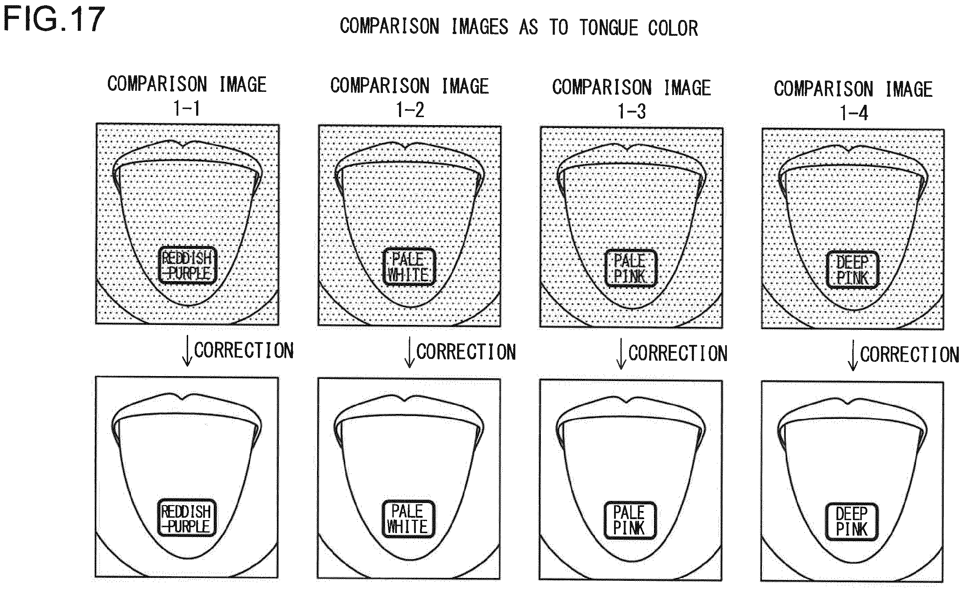

Fig. 17 schematically shows comparison images before and after correction in a case where a fluorescent lamp is used as the illumination light source at the time of acquisition of a reference image and a flash light source is used as the illumination light source at the time of acquisition of a shot image. Although the plurality of comparison images corrected by the image correction unit 62 are created based on the reference image obtained by shooting under a fluorescent lamp, their entire images have a color tone closer to that of the shot image (see Fig. 9) obtained by shooting under a flash light. The data of the plurality of comparison images thus corrected is transmitted to the degree-of-health outputting device 1 by the communication unit 65. Thus, with the degree-of-health outputting device 1, the user can see the plurality of comparison images having a color tone closer to that of the shot image and can easily select from them a comparison image showing a state of the tongue close to that shown in the shot image. That is, with the degree-of-health outputting device 1, even in a case where, when a shot image is acquired, the tongue is shot at different places where the ambient light has color temperatures different than at the time of acquisition of the reference image, it is possible to present the user with a plurality of comparison images with an appropriate color tone close to that of the shot image and let him select one of them with ease.

In a case where the illumination light at the time of acquisition of a shot image is the same as the illumination light at the time of acquisition of a reference image (in a case where they have the same color temperature), the processing for correcting comparison images by the image correction unit 62 may be omitted.

[Control Flow]

Next, the flow of operation in the degree-of-health outputting system 100 according to the embodiment will be described. The operation roughly divides into processing for creating comparison images and processing for determining the degree of health. These will now be described.

[Process for Creating Comparison Images]

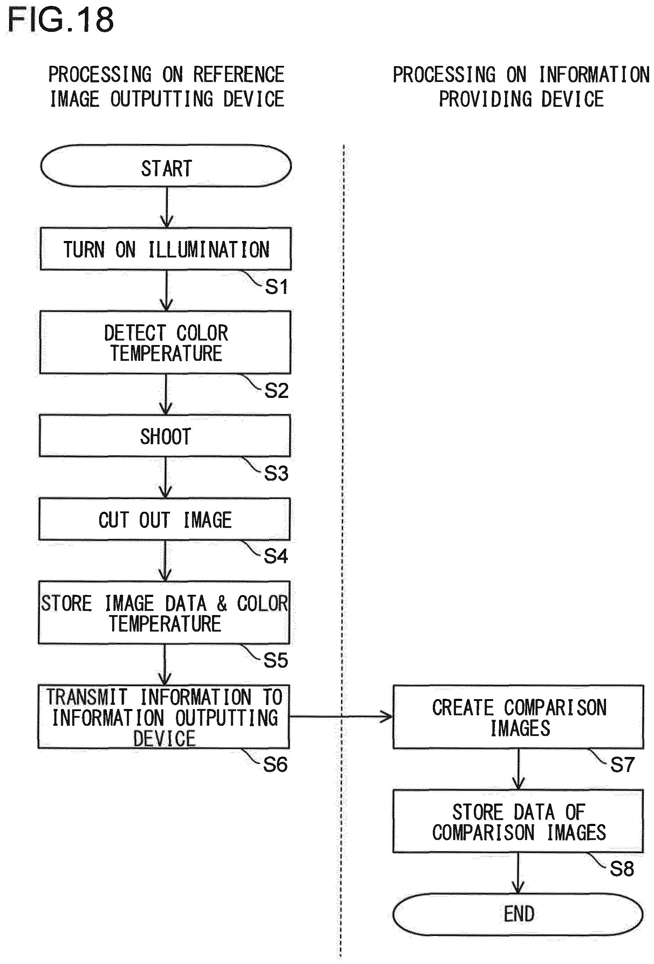

Fig. 18 is a flow chart showing the flow of the processing for creating comparison images. First, before the shooting of a reference image in the reference image outputting device 50, an illumination unit (for example, a fluorescent lamp) in the ambience is lit (S1), and the color temperature at that time is detected with a sensor (unillustrated) (S2). At S2, instead of the color temperature being detected with the sensor, white paper may be shot in the reference image imaging unit 53 to acquire RGB image data and this may be taken as information on the color temperature. Then, the face of a living subject is shot by the reference image imaging unit 53 (S3). At this time, the living subject is assumed to be in healthy condition (normal condition).

Subsequently, from the RGB image data of the face thus shot, a part (for example, the tongue) whose condition varies according to the degree of health is recognized, and an image including that part is cut out as a reference image (S4). The data of the image (reference image) thus cut out is stored in the storage unit 55 (S5). At this time, also information on the color temperature measured at S2 is stored in the storage unit 55.

Then, the information (the data of the reference image and the information on the color temperature) stored in the storage unit 55 is transmitted to the information providing device 60 by the communication unit 56 (S6). Thus, in the information providing device 60, by the comparison image creation unit 61 (S7), from the reference image included in the transmitted information, a plurality of comparison images are created by image processing. Here, it is assumed that the plurality of comparison images are created for each of four diagnosis items, namely tongue color, tongue shape (tooth marks), tongue coating color, and tongue coating thickness; instead, the plurality of comparison images may be created for only one diagnosis item. The data of the plurality of comparison images created for each diagnosis item is stored in the storage unit 63 (S8).

(Processing for Determining Degree of Health)

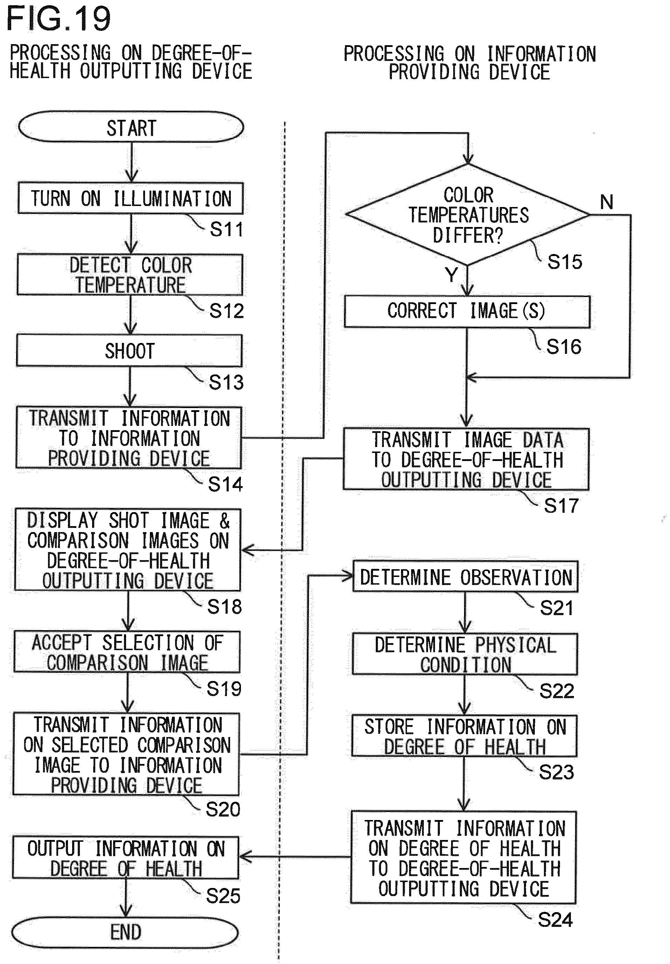

Next, the processing for determining the degree of health will be described. Fig. 19 is a flow chart showing the flow of the processing for determining the degree of health. First, before the shooting of an image in the degree-of-health outputting device 1, an illumination unit (for example, a flash light source) in the ambient is lit (S11), and the color temperature at that time is detected in the detection unit 6 (S12). At S12, instead of the color temperature being detected by the detection unit 6, white paper may be shot in the imaging unit 3 to obtain RGB image data and this may be taken as information on the color temperature.

Subsequently, a still image of the tongue is shot by the imaging unit 3 with respect to the same living subject as at the time of acquisition of the reference image (S13). For example, while a movie of the user's tongue is shot with the imaging unit 3, the movie is displayed on the display unit 4; while viewing the movie of his own tongue, the user can, by pressing the OK button 5a in the operation unit 5 with predetermined timing, obtain a still image of the tongue. At this time, a frame line that represents the shooting position of the tongue may be displayed on the display unit 4 to indicate the shooting position of the tongue. Then, the information on the color temperature of the ambient light at the time of acquisition of the shot image detected at step S12 and the data of the shot image acquired at S13 are transmitted to the information providing device 60 via the communication unit 7 (Step S14).

Next, the image correction unit 62 in the information providing device 60 checks whether or not the color temperature at the time of acquisition of the shot image transmitted from the degree-of-health outputting device 1 differs from the color temperature at the time of acquisition of the reference image transmitted from the reference image outputting device 50 (S15), and if they differ (for example, if the difference in color temperature is 100 K or more), performs the above-described correction on at least either the plurality of comparison images (the data of the comparison images stored in the storage unit 63) or the shot image (the data of the shot image transmitted from the degree-of-health outputting device 1) to bring the color tone closer together between the two (S16). Then, the communication unit 65 transmits the data of the image(s) corrected in the image correction unit 62 to the degree-of-health outputting device 1 (S17). On the other hand, if, at S15, the color temperature at the time of acquisition of the shot image and the color temperature at the time of acquisition of the reference image are approximately the same (for example, if the difference in color temperature is less than 100 K), the processing in step S16 is skipped. Then, at S 17, the communication unit 65 transmits the data of the plurality of comparison images stored in the storage unit 63 to the degree-of-health outputting device 1.

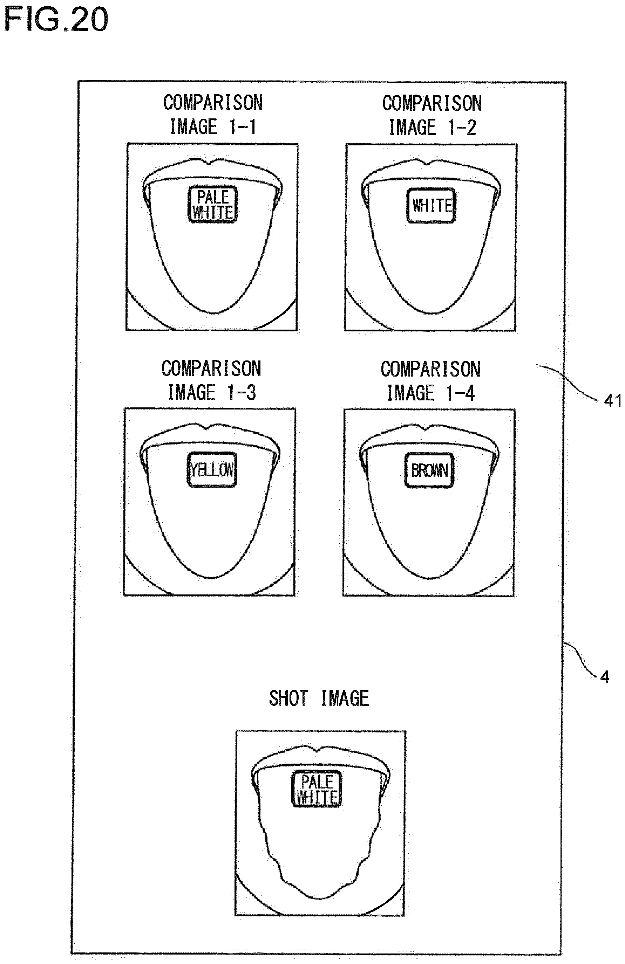

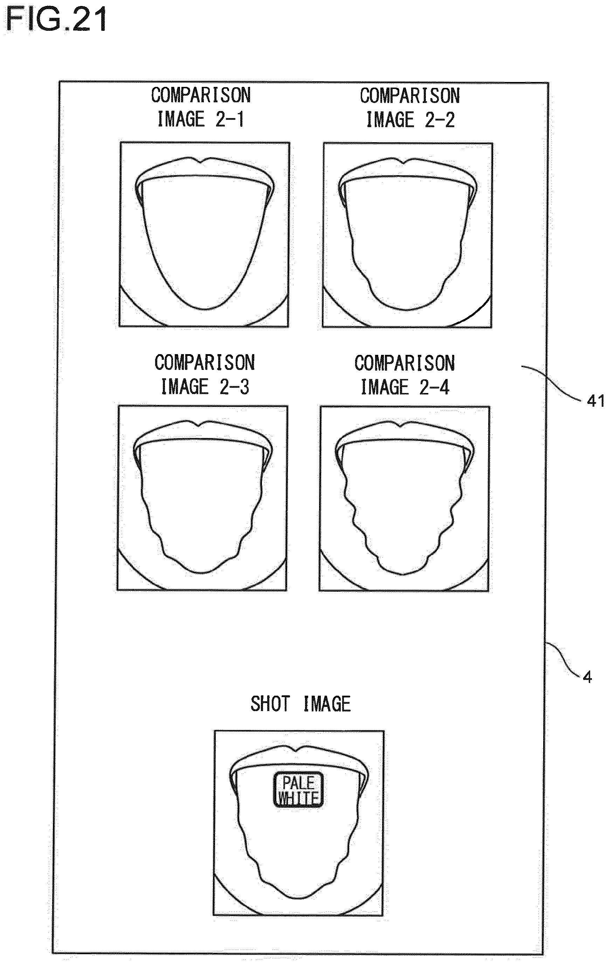

In the degree-of-health outputting device 1, the shot image and the plurality of comparison images are displayed together on the display unit 4 (S18). At this time, on the display unit 4, according to operation on the operation unit 5, the plurality of comparison images can be displayed switchably for each diagnosis item, or the plurality of comparison images can be displayed simultaneously for each diagnosis item. Fig. 20 schematically shows a state where the shot image and the plurality of comparison images are displayed together on the display unit 4 as to tongue coating color as one diagnosis item, and the Fig. 21 schematically shows a state where the shot image and the plurality of comparison images are displayed together on the display unit 4 as to tongue shape as one diagnosis item. With such display, the user can view the display unit 4, compare the shot image with the plurality of comparison images, and select a comparison image with a tongue coating color close to that in the shot image by pressing the operation unit 5 (touch-panel display device 41).

When the operation unit 5 accepts selection of a comparison image by the user for each diagnosis item (S19), information on the comparison image selected for each diagnosis item (for example, the number of the comparison image) is transmitted to the information providing device 60 via the communication unit 7 (S20). In the information providing device 60, the degree-of-health determination unit 64 refers to the table stored in the storage unit 63, and extracts, for each diagnosis item, the degree of health corresponding to the selected comparison image. That is, the degree-of-health determination unit 64 determines the degree of health (determines an observation) for each diagnosis item, as by determining the tongue coating color as "level 1", determining the tongue shape as "level 3", and so forth (S21).

Subsequently, based on the observation determined for each diagnosis item, the degree-of-health determination unit 64 determines the physical condition of the examinee in the light of criteria for *Kampo* diagnosis (S22). For example, as to tongue coating color, when the degree of health is "level 1", it is determined to indicate a normal condition; as to tongue shape, when the degree of health is "level 3", it is determined to indicate "abnormal water metabolism". These determination results are, as information as to the degree of health, stored in the storage unit 63 (S23) and also transmitted from the communication unit 65 to the degree-of-health outputting device 1 (S24). Thus, in the degree-of-health outputting device 1, the display unit 4 outputs information (such as abnormal water metabolism) as to the degree of health (S25). At S25, information as to the degree of health may be audibly outputted from the audio output unit 8, or display on the display unit 4 and audio output from the audio output unit 8 may be performed simultaneously.

As described above, on the display unit 4 are displayed a shot image of a part (in the above example, the tongue) whose condition varies according to the degree of health in a living subject and a plurality of comparison images with different degrees of health (see Figs. 20 and 21). The shot image and the plurality of comparison images all relate to the same living subject, and thus the information outputted on the output unit 5 excludes the influence of individual differences from other living subjects. Thus, the user can grasp accurately the degree of health of the living subject. Moreover, the plurality of comparison images are created by image processing, and even conditions that fall beyond the range of variation of the examinee's health condition in the past can be presented as comparison images; thus, even in a case where the examinee's health condition at the time of diagnosis falls beyond the range of variation of his health condition in the past, by selecting a comparison image and outputting the degree of health based on it, it is possible to grasp accurately the examinee's degree of health.

In the embodiment, the reference image is an image shot, with respect to the same single living subject, when it is healthy. In this case, the plurality of comparison images created from the reference image are created with reference to a healthy condition, and thus the user can, by comparing the shot image with the plurality of comparison images on the display unit 4, grasp easily a subtle change in physical condition from the healthy condition (whether or not in an initial stage of a disease).

The plurality of comparison images are images that are obtained by varying the reference image over a plurality of grades with different degrees of health for at least one diagnosis item. By displaying a plurality of comparison images for at least one diagnosis item and permitting selection of one of them on the operation unit 5, it is possible to output, based on the selected comparison image, information as to the degree of health of the living subject for at least one diagnosis item.

The part whose condition varies according to the degree of health is the tongue of the living subject, and the above-mentioned diagnosis items include tongue color, tongue shape, tongue coating color, and tongue coating thickness. Thus, it is possible to output, based on a comparison image selected for at least one of those diagnosis items, information as to the degree of health of the living subject.

The display unit 4 displays a plurality of comparison images created for a plurality of diagnosis items in a manner corresponding to each diagnosis item, and outputs information as to the degree of health of a living subject based on the comparison image selected on the operation unit 5 for each diagnosis item. In this case, the user can grasp the degree of health corresponding to each diagnosis item based on the outputted information.

The information providing device 60 includes a comparison image creation unit 61, which creates a plurality of comparison images from a reference image by image processing, and a communication unit 65, which outputs the data of the plurality of comparison images to the degree-of-health outputting device 1. Thus, the degree-of-health outputting device 1 can receive the data of the plurality of comparison images created in the information providing device 60, display the plurality of comparison images on the display unit 4, and let the user select one of the comparison images on the operation unit 5. Thus, the degree-of-health outputting device 1 does not need to be provided with a unit (circuit) for creating comparison images, and this helps simplify the configuration of the degree-of-health outputting device 1.

Even in a case where the type of illumination light differs between at the time of acquisition of the shot image and at the time of acquisition of the reference image, and thus the color tone of the entire image differs between the shot image and the plurality of comparison images, the image correction unit 62 in the information providing device 60 corrects at least either the shot image or the plurality of comparison images such that the chromaticity of the entire image of each comparison image is closer, in relative terms, to the chromaticity of the entire image of the shot image. Thus, even in a case where the type of illumination light differs between at the time of acquisition of the shot image and at the time of acquisition of the reference image, it is possible to bring the color tone of the entire image close together between the shot image and the plurality of comparison images. Thus, by transmitting the data of the corrected image(s) to the degree-of-health outputting device 1 via the communication unit 65, it is possible to permit the user easy selection of an appropriate comparison image close to the shot image out of the plurality of comparison images on the degree-of-health outputting device 1.

In particular, the image correction unit 62 corrects at least either the shot image or the plurality of comparison images such that the average value of the image data of each color (RGB) in each comparison image is closer, in relative terms, to the average value of the image data of each color in the shot image; thus, even in a case where the type of illumination light differs between at the time of acquisition of the shot image and at the time of acquisition of the reference image, it is possible to reliably bring the color tone of the entire image closer together between the shot image and the plurality of comparison images.

The degree-of-health determination unit 64 in the information providing device determines the degree of health of the living subject based on the comparison image selected on the operation unit 5 in the degree-of-health outputting device 1, and the communication unit 65 transmits information on the degree of health determined by the degree-of-health determination unit 64 to the degree-of-health outputting device 1. Thus, the degree-of-health outputting device 1 can receive the transmitted information on the degree of health and output it in the output unit (for example, display it on the display unit 4). Accordingly, the degree-of-health outputting device 1 does not need to be provided with a unit (circuit) for determining the degree of health, and this helps simplify the configuration of the degree-of-health outputting device 1.

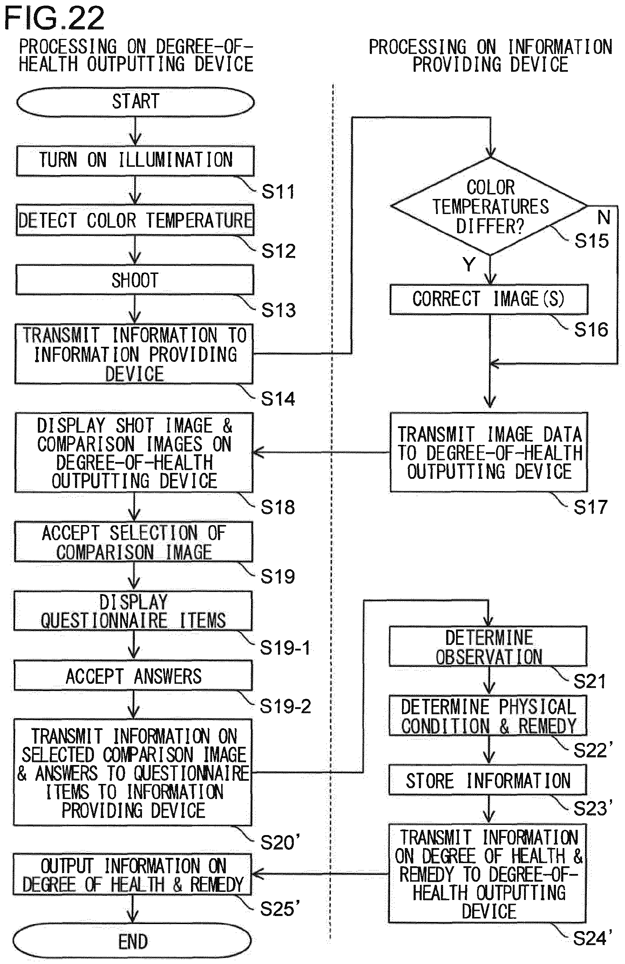

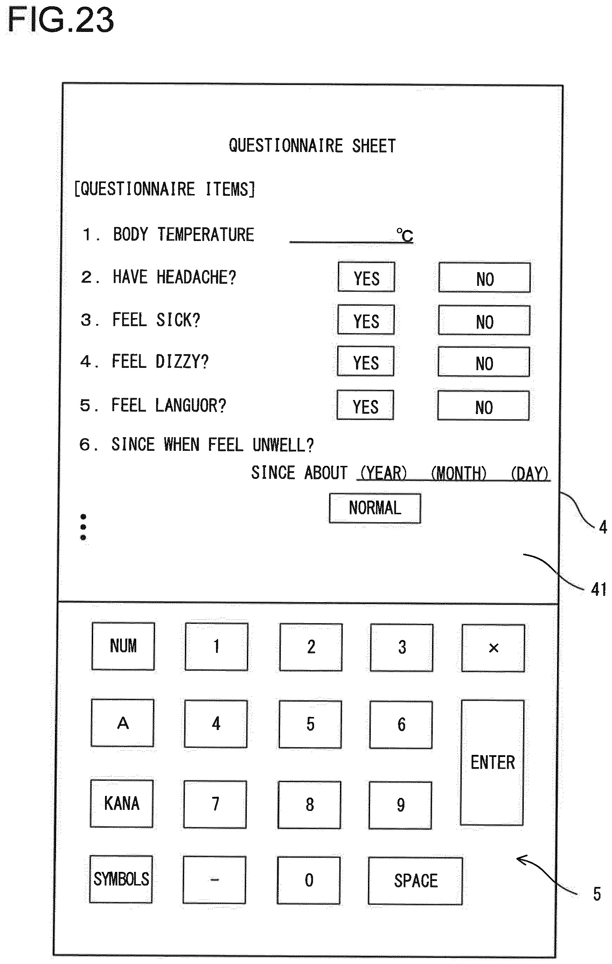

S19 (acceptance of selection of a comparison image) in Fig. 19 may be followed, instead, by processing as described below. Fig. 22 is a flow chart showing the flow of other processing for determining the degree of health. When, at S19, the operation unit 5 accepts selection of a comparison image, the display unit 4 displays questionnaire items as to the living subject (S19-1). Fig. 23 shows an example of a screen displayed when questionnaire items are displayed on the display unit 4. The questionnaire items include, for example, body temperature, whether subjective symptoms (headache, nausea, dizziness, and languor) are present, when subjective symptoms occur, and the like.

When questionnaire items as to the living subject are displayed on the display unit 4 as described above, the operation unit 5 functions as an input unit for accepting answers to the questionnaire items displayed on the display unit 4. For example, by displaying, along with the above-mentioned questionnaire items, software keys (keys for entering numbers, characters, and the like) as an input unit on the touch-panel display device 41, it is possible to permit the user to enter answers to the questionnaire items by pressing those software keys. When the operation unit 5 accepts entry of answers to the questionnaire items (S19-2), information on the comparison image selected at S19 (the number of the selected comparison image) and information on the answers to the questionnaire items entered at S19-2 are transmitted to the information providing device 60 via the communication unit 7 (S20').

In the information providing device 60, the degree-of-health determination unit 64 extracts, by referring to the table stored in the storage unit 63, the degree of health corresponding to the selected comparison image for each diagnosis item, and determines the degree of health (determines an observation) for each diagnosis item (S21). Subsequently, based on the observation determined for each diagnosis item, the degree-of-health determination unit 64 determines the physical condition (degree of health) of the examinee in the light of criteria for *Kampo* diagnosis, and determines a remedy that suits the determination result and the answers to the questionnaire items (S22'). For example, in a case where it is known, from empirical evidence in *Kampo* medicine, that an examinee with poor blood circulation combined with a subjective symptom of headache is recommended to take herbal medicine A, and that an examinee with abnormal water metabolism combined with prolonged languor is recommended to take herbal medicine B, then according to this knowledge, the degree-of-health determination unit 64 determines a remedy (for example, a herbal medicine to be taken). Based on the physical condition and the answers to the questionnaire items, not only a herbal or pharmaceutical medicine but also improvements on diet and lifestyle may be determined and presented.

The examinee's physical condition (information as to his degree of health) and the remedy are stored in the storage unit 63 (S23'), and are also transmitted from the communication unit 65 to the degree-of-health outputting 1 (S24'). Thus, in the degree-of-health outputting device 1, the display unit 4 outputs not only information as to the degree of health (poor blood circulation, abnormal water metabolism, and the like) but also a remedy (S25').

The answers to the questionnaire items reflect the subjective symptoms of the living subject himself as the target of diagnosis, and thus by taking those subjective symptoms of the examinee into consideration, it is possible to present an appropriate remedy. Moreover, owing to the display unit 4 displaying questionnaire items as to the living subject and outputting (displaying), along with information as to the degree of health, a remedy that suits the degree of health of the living subject and the answers to the questionnaire items entered on the operation unit 5, the user can not only grasp the outputted degree of health but also try to improve the degree of health based on the outputted remedy.

When answers to questionnaire items as to a living subject are entered on the degree-of-health outputting device 1, and information on them is transmitted to the information providing device 60, the degree-of-health determination unit 64 in the information providing device 60 determines a remedy that suits the determined degree of health of the living subject and the answers to the questionnaire items, and the communication unit 65 transmits information on the degree of health and the remedy determined in the degree-of-health determination unit 64 to the degree-of-health outputting device 1. Thus, the degree-of-health outputting device 1 can be configured to receive information on the degree of health and the remedy and output them in the output unit; thus, the degree-of-health outputting device 1 no longer needs to be provided with a unit for determining a remedy, and this helps simplify the configuration of the degree-of-health outputting device 1.

In a case where the information providing device 60 includes the comparison image creation unit 61 and the storage unit 63 described above and the storage unit 63 stores the plurality of comparison images created in the comparison image creation unit 61 along with a table defining the correspondence between each of the plurality of comparison images and information as to the degree of health, S17 and S18 in Fig. 19 may comprise processing as described below. At S17, the communication unit 65 transmits the data of the plurality of comparison images stored in the storage unit 63 and information on the degree of health of the living subject corresponding to each of the comparison images to the degree-of-health outputting device 1. In the degree-of-health outputting device 1, the display unit 4 displays the shot image, the plurality of comparison images, and information on the degree of health corresponding to each of the comparison images (S18). Fig. 24 shows one example of a screen displayed on the display unit 4 at S18. In this way, in a case where a plurality of comparison images are displayed along with information on the degree of health of the living subject corresponding to each of the comparison images, the processing at S 19 and the following steps may be omitted.

As described above, on the display unit 4 are displayed a shot image of a part (in the above example, the tongue) whose condition varies according to the degree of health in a living subject, a plurality of comparison images with different degrees of health, and information on the degree of health of the living subject corresponding to each of the comparison images (see Fig. 24). Thus, the user can, by viewing the shot image of the living subject at present and information on the degree of health displayed to correspond to the comparison image closest to that image, grasp the degree of health of the user himself accurately and easily.

Based on what has been described above, it can be said that the degree-of-health outputting device 1 according to the embodiment may include a display unit 4 that displays a shot image obtained by shooting a part whose condition varies according to the degree of health in a living subject and that together displays a plurality of comparison images with different degrees of health obtained based on a reference image (created by image processing from a reference image) obtained by shooting the same part before the time of acquisition of the shot image with respect to the same living subject along with information as to the degree of health of the living subject corresponding to each of the plurality of comparison images.

It can also be said that the degree-of-health outputting system 100 includes the degree-of-health outputting device 1 and the information providing device 60 described above, and that the information providing device 60 may include a comparison image creation unit 61 which creates the plurality of comparison images from the reference image by image processing, a storage unit 63 which stores a table defining the correspondence between each of the comparison images and information as to the degree of health, and a communication unit 65 which outputs the data of the plurality of comparison images and the information as to the degree of health corresponding to each of the plurality of comparison images to the degree-of-health outputting device 1.

[Another Configuration of the Degree-of-Health Outputting System]

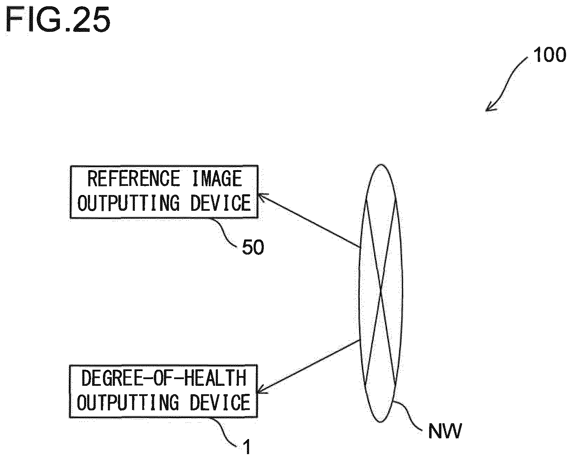

Fig. 25 is an explanatory diagram showing another configuration of the degree-of-health outputting system 100. In this degree-of-health outputting system 100, the degree-of-health outputting device 1 described above doubles to function also as the information providing device 60.

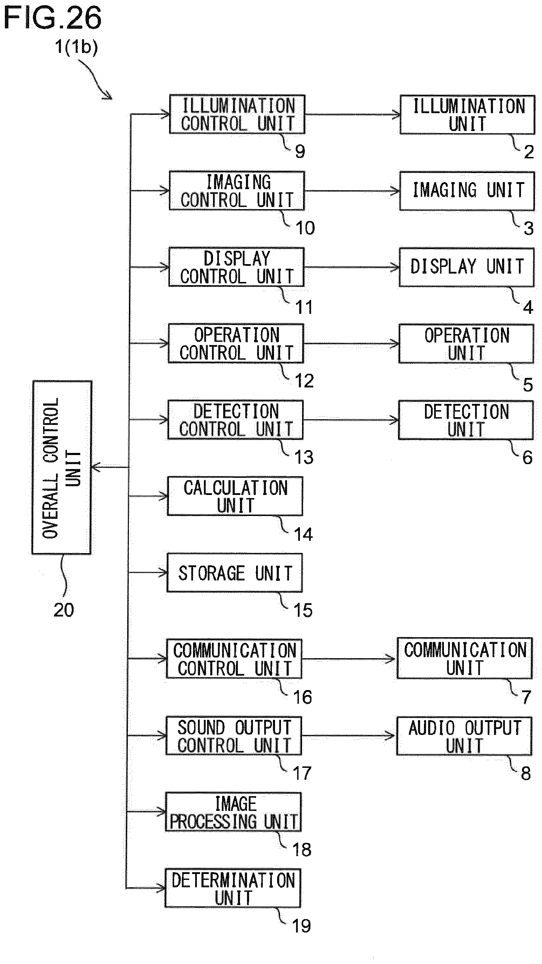

Fig. 26 is a block diagram showing an outline configuration of an organ image shooting device 1b as a degree-of-health outputting device 1 in the degree-of-health outputting system 100 in Fig. 25. Compared with the configuration of the organ image shooting device 1a shown in Fig. 3, the organ image shooting device 1b further includes an image processing unit 18 and a determination unit 19. The image processing unit 18 is a processing circuit for creating a plurality of comparison images from a reference image by image processing, and corresponds to the comparison image creation unit 61 in the information providing device 60 in Fig. 7. Accordingly, the image processing unit 18 can create a plurality of comparison images by a method similar to that which the comparison image creation unit 61 adopts.

The image processing unit 18 also functions as a correction unit that, when the color temperature at the time of acquisition of the shot image detected by the detection unit 6 differs from the color temperature at the time of acquisition of the reference image transmitted from the reference image outputting device 50, corrects at least either the shot image or the plurality of comparison images such that the color tone of the entire image of each of the comparison image is closer, in relative terms, to the color tone of the entire image of the shot image. The image processing unit 18 as the correction unit corresponds to the image correction unit 62 in the information providing device 60 in Fig. 7. Accordingly, the image processing unit 18 can correct at least either the shot image or the plurality of comparison images by a method similar to that which the image correction unit 62 adopts.

The determination unit 19 is a processing circuit for determining the degree of health of the living subject based on the comparison image selected on the operation unit 5 out of the plurality of comparison images displayed on the operation unit 5, and corresponds to the degree-of-health determination unit 64 in the information providing device 60 in Fig. 7. Storing a table defining the relationship between the plurality of comparison images and the degrees of health in the storage unit 15 enables the determination unit 19 to refer to the table stored in the storage unit 15 and extract and determine, for each diagnosis item, the degree of health corresponding to the selected comparison image.

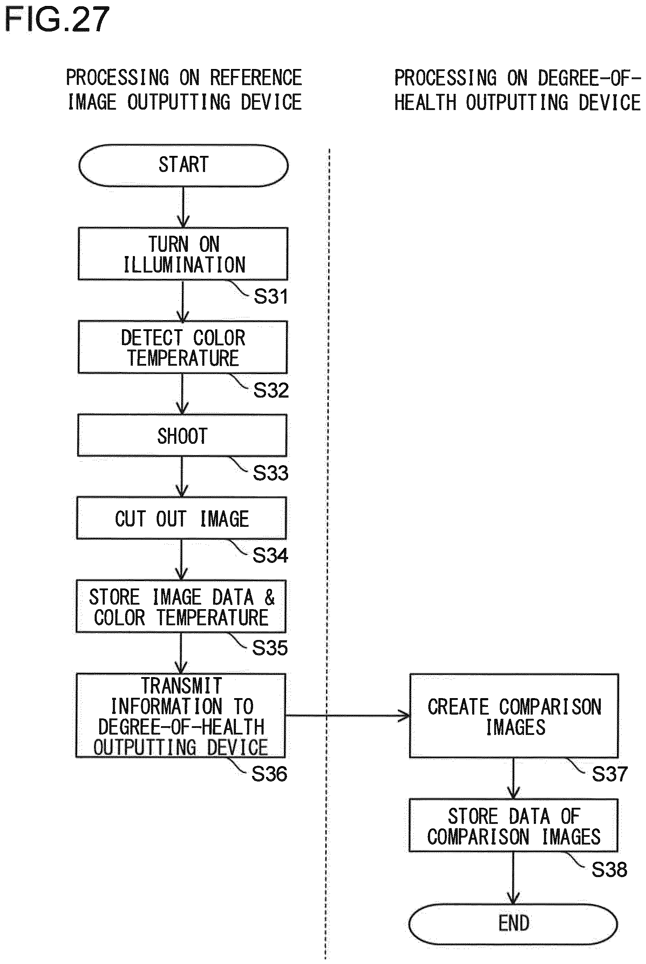

Fig. 27 is a flow chart showing the flow of operation for creating comparison images in the degree-of-health outputting system 100 described above. The processing at S31 through S35 is quite similar to the processing at S1 through S5 in Fig. 18. At S36, the transmission destination of the image data of the reference image and the information on the color temperature stored at S35 is the degree-of-health outputting device 1 (organ image shooting device 1b), and in this respect S36 differs from S6 in Fig. 18. S37 and S38 are processing performed on the degree-of-health outputting device 1. That is, at S37, by the image processing unit 18 in the degree-of-health outputting device 1, from the reference image included in the information transmitted from the reference image outputting device 50, the plurality of comparison images are created for each diagnosis item by image processing. The data of the plurality of comparison images created for each diagnosis item is stored in the storage unit 15 (S38).

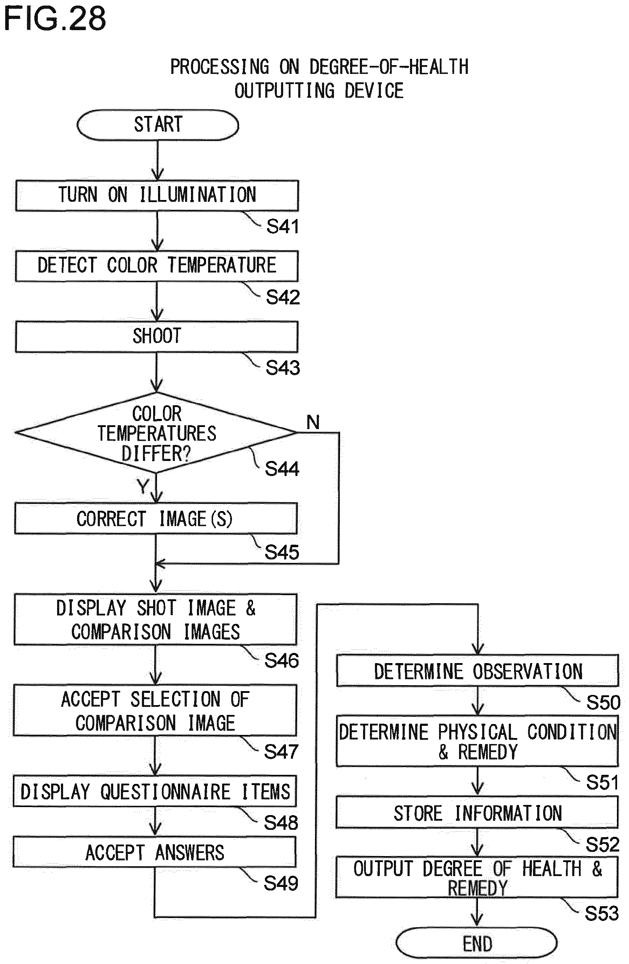

Fig. 28 is a flow chart showing the flow of processing for determining the degree of health in the degree-of-health outputting system 100 described above. The processing shown in Fig. 28 lacks the step of transmitting and receiving information between the degree-of-health outputting device 1 and the information providing device 60, and is basically the same as the processing shown in Fig. 22 except that the whole processing is performed principally by the degree-of-health outputting device 1. How the individual steps in Fig. 28 correspond to those in Fig. 22 is as follows.

The processing at S41 through S43 in Fig. 28 is quite similar to that at S11 through S13 in Fig. 22, and the processing at S44 and S45 is quite similar to that at S15 and S16 described above. However, the processing for image correction at S44 and S45 is performed principally by the image processing unit 18.

The processing at S46 through S49 is quite similar to that at S18, S19, S19-1, and S19-2, and the processing at S50 through S52 is quite similar to that at S21, S22', and S23' described above. However, the determination at S50 and S51 is principally performed by the determination unit 19, and at S52, the determination result is stored in the storage unit 15. The processing at S53 is quite similar to that at S25' described above.

As described above, the degree-of-health outputting device 1 includes the image processing unit 18 which creates a plurality of comparison images from a reference image by image processing and the determination unit 19 which determines the degree of health of a living subject based on the comparison image selected on the operation unit 5. Then, the output unit (for example, the display unit 4) outputs information as to the degree of health determined by the determination unit 19. By furnishing the degree-of-health outputting device 1 with the functions of the information providing device 60 (the functions for creating comparison images and for determining the degree of health and the like) in this way, it is possible to determine and output the degree of health with the reference image outputting device 50 and the degree-of-health outputting device 1 connected together such that these can communicate directly with each other. Thus, the information providing device 60 no longer needs to be interposed, and this helps simplify the configuration of the degree-of-health outputting system 100. Moreover, because no connection for communication with the information providing device 60 is necessary, in a case where the communication charge for the degree-of-health outputting device 1 depends on the amount of packets exchanged during data communication, the communication charge can be saved.

The image processing unit 18 in the degree-of-health outputting device 1, like the image correction unit 62 in the information providing device 60, functions also as a correction unit which, when the color temperature at the time of acquisition of the shot image differs from the color temperature at the time of acquisition of the reference image, corrects at least either the shot image or the plurality of comparison images such that the chromaticity of the entire image of each of the comparison images is closer, in relative terms, to the chromaticity of the entire image of the shot image. Thus, even in a case where the illumination light differs between at the time of acquisition of the shot image and at the time of acquisition of reference image, it is possible to bring the color tone of the entire image closer together between the shot image and the plurality of comparison images to permit the user to easily select an appropriate comparison image close to the shot image out of the plurality of comparison images. In particular, the image processing unit 18 corrects at least either the shot image or the plurality of comparison images such that the average value of the image data of each color in each of the comparison images is closer, in relative terms, to the average value of the image data of each color in the shot image; thus, it is possible to reliably bring the tone color of the entire image closer together between the shot image and the plurality of comparison images.

The degree-of-health outputting device 1 shown in Fig. 26 can be used as the degree-of-health outputting device 1 in the degree-of-health outputting system 100 in Fig. 1.

[Other]

Although the above description deals with a case where the living subject is a human, the living subject may be an animal other than a human. For example, even with the tongue of an animal, such as a pet or farm animal, it is possible to apply a method according to the embodiment to output the degree of health. In that case, the user can quickly and appropriately determine an unhealthy condition of an animal, which is incapable of verbal communication.

Although in the above description an image shot when a living subject himself is healthy is used as a reference image based on which a plurality of comparison images are created, instead an image shot when a living subject himself is unhealthy (diseased) may be used. Even in that case, out of a plurality of comparison images created with reference to an unhealthy condition, one close to the part shown in a current shot image can be selected to grasp the degree of health. Instead, as reference images, one shot in healthy condition and one shot in diseased condition may both be used, and these may be included in comparison images. How the tongue is diagnosed (how the degree of health changes) varies from one individual to another, and thus grasping conditions at opposite ends, namely healthy and diseased conditions, enables presentation of more accurate comparison images that suit an individual living subject.

Although the above description deals with an example where the tongue is taken as a part whose condition changes according to the degree of health in a living subject, any part other than the tongue, such as an internal organ (for example, the inner lining of the large intestine) or a part on the body surface (for example, the face or the eyelid), may instead be taken so long as its condition (color and shape) changes according to the degree of health; to that part, it is possible to apply a method according to the embodiment to grasp the degree of health.

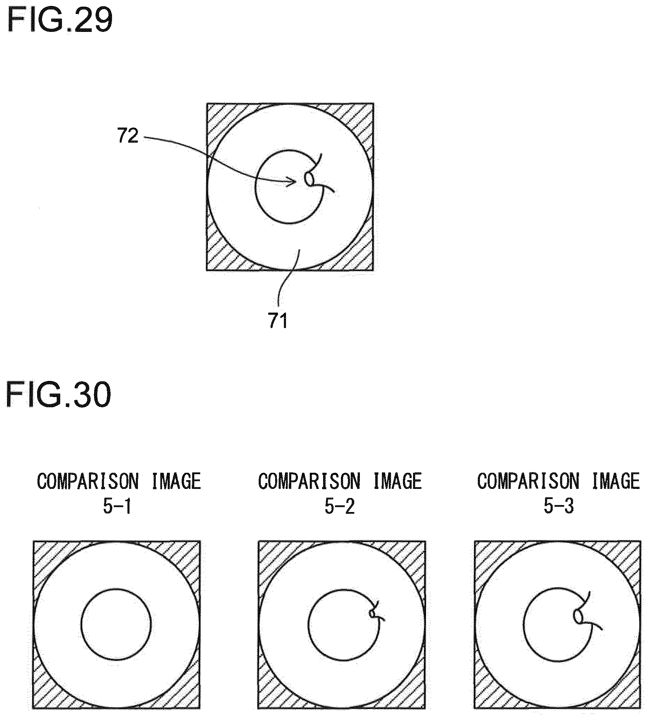

For example, Fig. 29 schematically shows an image obtained by observing the inner lining 71 of the large intestine as one example of human body tissues with the endoscope. There, the inner lining 71 exhibits an ulcerous projection 72. The contour of this projection 72 may be extracted to create a plurality of comparison images 5-1 to 5-3 as shown in Fig. 30 by varying its shape from large to small as with tooth marks. Also in that case, by displaying on the display unit 4 a shot image that is currently being observed and shot with an endoscope along with the plurality of comparison images created, letting the user (in this case, a doctor) select an appropriate comparison image, and outputting information on the degree of health based on the selected comparison image, it is possible to permit the user to grasp the degree of health of the living subject and make a diagnosis while taking the outputted information into consideration.

Although the above description deals with a configuration where a reference image based on which a plurality of comparison images are created is acquired in the reference image outputting device 50 and its data is transmitted to the information providing device 60, a configuration is also possible where the degree-of-health outputting device 1 acquires the reference image with the imaging unit 3 and transmits its data to the information providing device 60.

Thus, the degree-of-health outputting device 1 and the degree-of-health outputting system 100 according to the embodiment can not only assist less-experienced doctors and medical professionals such nurses, care workers, and pharmacists but also, as far as skin and mucous membrane on the body surface and the like concern, permit patients themselves to make a simplified diagnosis by using the degree-of-health outputting device 1, and this is expected to be useful in day-to-day management of physical condition and early discovery of diseases.

As described above, according to one embodiment, a degree-of-health outputting device, includes: a display unit configured to display together a shot image obtained by shooting a part whose condition changes according to the degree of health in a living subject and a plurality of comparison images with different degrees of health obtained based on a reference image (created (acquired) from a reference image by image processing) obtained by shooting the part before the time of acquisition of the shot image with respect to the same living subject; an operation unit configured to be operated to select one of the plurality of comparison images displayed on the display unit; and an output unit configured to output information as to the degree of health of the living subject based on the comparison image selected on the operation unit.

With the above configuration, on the display unit, with respect to the same living subject, a shot image of a predetermined part (for example, the tongue) and a plurality of comparison images with different degrees of health (levels of disease) created by image processing are displayed. The plurality of comparison images may be created on the degree-of-health outputting device, or may be created on an external device (for example, a server) and transmitted to the degree-of-health outputting device. Thus, the user can easily grasp which of the plurality of comparison images shows the condition to which the condition of the part shown in the shot image is closest, and can select the closest comparison image on the operation unit. When a comparison image is selected on the operation unit, based on the selected comparison image, information as to the degree of health of the living subject is outputted (for example, displayed or audibly outputted) in the output unit.

Thus, the shot image and the targets (the plurality of comparison images) with which it is compared are ones as to the same living subject, and accordingly the outputted degree of health excludes the influence of an individual difference from other living subjects. This enables the user to grasp accurately the degree of health (level of disease) of the living subject. In addition, the plurality of comparison images are obtained based on the reference image (created by image processing), and even a condition that falls outside the range of variation of the examinee's health condition in the past can be presented as a comparison image; thus, even if the examinee's health condition at the time of diagnosis falls outside the range of variation of physical condition in the past, by selecting a comparison image and outputting the degree of health based on it, it is possible to grasp accurately the examinee's degree of health.

The reference image may be an image shot in healthy condition with respect to the same living subject. The plurality of comparison images with different degrees of health are created with reference to a healthy condition. Thus, by comparing the shot image with the plurality of comparison images on the display unit, the user can grasp easily a subtle change from the healthy condition (whether or not in an initial stage of a disease).

The plurality of comparison images may be images obtained by changing the reference image over a plurality of grades with different degrees of health with respect to at least one diagnosis item that serves as an index in determining the degree of health of the living subject. In this case, it is possible to output the information as to the degree of health of the living subject on the output unit based on the comparison image selected for one diagnosis item, or based on the comparison image selected for each diagnosis item.

The part may be the tongue of the living subject, and the diagnosis item may include at least one of tongue color, tongue shape, tongue coating color, and tongue coating thickness. In this case, it is possible to output the information as to the degree of health of the living subject on the output unit based on a comparison image selected with respect to at least one of tongue color, tongue shape, tongue coating color, and tongue coating thickness.

The display unit may be configured to display, for each of a plurality of diagnosis items, the plurality of comparison images obtained with respect to that diagnosis item, and the output unit may configured to output, for each of the plurality of diagnosis items, the information as to the degree of health of the living subject based on the comparison image selected for that diagnosis item on the operation unit. In this case, the user can grasp the degree of health corresponding to each diagnosis item based on the outputted information.

The display unit may be configured to further display a questionnaire item as to the living subject, the degree-of-health outputting device may further include an input unit configured to accept input of an answer to the questionnaire item displayed on the display unit, and the output unit may be configured to output, along with the information as to the degree of health, a remedy suitable for the outputted degree of health of the living subject and the answer inputted on the input unit.

An answer to a questionnaire item reflects a subjective symptom of the living subject (for example, headache, languor, and the like), and with such a subjective symptom taken into consideration, it is possible to present an appropriate remedy as by presenting an appropriate herbal medicine or alimentary therapy. Thus, the user can not only grasp the outputted degree of health but also, based on the outputted remedy, easily try to improve the degree of health.

The degree-of-health outputting device described above may further include an image processing unit configured to create the plurality of comparison images from the reference image by image processing. It is then possible to obtain the above effects with a configuration where the degree-of-health outputting device itself includes an image processing unit that creates the plurality of comparison images.

The degree-of-health outputting device described above may further include a determination unit configured to determine the degree of health of the living subject based on the comparison image selected on the operation unit, and the output unit may be configured to output information as to the degree of health determined by the determination unit. It is then possible to obtain the above effects with a configuration where the degree-of-health outputting device itself determines and outputs the degree of health of the living subject.

As described above, according to another embodiment, a program makes a computer execute: a process of displaying, on a display unit, a shot image obtained by shooting a part whose condition changes according to the degree of health in a living subject and a plurality of comparison images with different degrees of health obtained based on a reference image obtained by shooting the part before the time of acquisition of the shot image with respect to the same living subject; and a process of, when one of the plurality of comparison images displayed on the display unit is selected on an operation unit, outputting, from an output unit, information as to the degree of health of the living subject based on the selected comparison image. By making the computer execute the program, it is possible to make the computer function as a degree-of-health outputting device as described above.

As described above, according to another embodiment, a degree-of-health outputting system includes: a degree-of-health outputting device as described above; and an information providing device. Here, the information providing device includes: a comparison image creation unit configured to create the plurality of comparison images from the reference image by image processing; and a communication unit configured to output data of the plurality of comparison images to the degree-of-health outputting device.

With the above system, the degree-of-health outputting device can receive the data of a plurality of comparison images created on the information providing device, display the plurality of comparison images on the display unit, and allow selection of one of the comparison images on the operation unit. This helps reduce the burden of processing for creating the comparison images on the degree-of-health outputting device. The data of the reference image may be transmitted from the degree-of-health outputting device to the information providing device, or may be transmitted from another device (for example, a personal computer) to the information providing device.

The degree-of-health outputting system described above may further include: a reference image outputting device configured to transmit the data of the reference image and information as to the color temperature of the ambient light at the time of acquisition of the reference image to the information providing device. The degree-of-health outputting device may include: an imaging unit configured to acquire the shot image by shooting the part of the living subject; an illumination unit configured to illuminate the part of the living subject at the time of shooting by the imaging unit; a detection unit configured to detect the color temperature of light around the part illuminated by the illumination unit; and a communication unit configured to transmit the image data of the shot image acquired by the imaging unit and information on the color temperature detected by the detection unit to the information providing device. The information providing device may further include: an image correction unit configured to correct at least either the shot image or the plurality of comparison images such that the chromaticity of the entire image of each of the comparison images is closer, in relative terms, to the chromaticity of the entire image of the shot image when the color temperature at the time of acquisition of the shot image transmitted from the degree-of-health outputting device differs from the color temperature at the time of acquisition of the reference image transmitted from the reference image outputting device. The communication unit in the information providing device may be configured to transmit the data of the image or images corrected by the image correction unit to the degree-of-health outputting device.

If the type of illumination light (light from a fluorescent lamp, light from an incandescent lamp, light from a camera flash, and the like) differs between at the time of acquisition of the shot image and at the time of acquisition of the reference image based on which the plurality of comparison images are created, the chromaticity (color tone) of the entire image may differ between the shot image and the plurality of comparison images, and, in the degree-of-health outputting device, this may make it difficult to select on the operation unit a comparison image that shows a condition of the part of the living subject similar to the condition shown in the shot image. By correcting as described above at least either the shot image or the plurality of comparison images in the image correction unit in the information providing device, it is possible to bring the color tone of the entire image closer together between the shot image and the plurality of comparison images. Then, by transmitting the data of the corrected image(s) from the information providing device to the degree-of-health outputting device, in the degree-of-health outputting device, even if the illumination light differs between at the time of acquisition of the shot image and at the time of acquisition of the reference image, it is possible to allow the user select more easily an appropriate comparison image close to the shot image out of the plurality of comparison images.

The image correction unit in the information providing device may be configured to correct at least either the shot image or the plurality of comparison images such that the average value of the image data of each color in each of the comparison images is closer, in relative terms, to the average value of the image data of each color in the shot image. Through such correction, it is possible to reliably bring the color tone of the entire image closer together between the shot image and the plurality of comparison images.

The degree-of-health outputting system described above may further include: a reference image outputting device configured to transmit the data of the reference image and information as to the color temperature of the ambient light at the time of acquisition of the reference image to the information providing device. The communication unit in the information providing device may be configured to transmit the information on the color temperature at the time of acquisition of the reference image transmitted from the reference image outputting device along with the data of the plurality of comparison images to the degree-of-health outputting device. The degree-of-health outputting device may include: an imaging unit configured to acquire the shot image by shooting the part of the living subject; an illumination unit configured to illuminate the part of the living subject at the time of shooting by the imaging unit; a detection unit configured to detect the color temperature of the light around the part illuminated by the illumination unit; and a correction unit configured to correct at least either the shot image or the plurality of comparison images such that the chromaticity of the entire image of each of the comparison images is closer, in relative terms, to the chromaticity of the entire image of the shot image when the color temperature at the time of acquisition of the shot image detected by the detection unit differs from the color temperature at the time of acquisition of the reference image transmitted from the information providing device.

If the type of illumination light (light from a fluorescent lamp, light from an incandescent lamp, light from a camera flash, and the like) differs between at the time of acquisition of the shot image and at the time of acquisition of the reference image based on which the plurality of comparison images are created, the chromaticity (color tone) of the entire image may differ between the shot image and the plurality of comparison images, and, in the degree-of-health outputting device, this may make it difficult to select on the operation unit a comparison image that shows a condition of the part of the living subject similar to the condition shown in the shot image. By correcting as described above at least either the shot image or the plurality of comparison images in the correction unit in the degree-of-health outputting device, it is possible to bring the color tone of the entire image closer together between the shot image and the plurality of comparison images. Thus, even if the illumination light differs between at the time of acquisition of the shot image and at the time of acquisition of the reference image, it is possible to allow the user select more easily an appropriate comparison image close to the shot image out of the plurality of comparison images.

The correction unit in the degree-of-health outputting device may be configured to correct at least either the shot image or the plurality of comparison images such that the average value of the image data of each color in each of the comparison images is closer, in relative terms, to the average value of the image data of each color in the shot image. Through such correction, it is possible to reliably bring the tone color of the entire image closer together between the shot image and the plurality of comparison images.

The information providing device may further include: a degree-of-health determination unit configured to determine the degree of health of the living subject based on the comparison image selected on the operation unit in the degree-of-health outputting device, and the communication unit may be configured to transmit information on the degree of health determined by the degree-of-health determination unit to the degree-of-health outputting device.

The degree-of-health outputting device can receive information on the degree of health of the living subject determined by the information providing device, and output it on the output unit. This helps reduce the burden of processing for determining the degree of health on the degree-of-health outputting device.

The degree-of-health determination unit may be configured to determine, when an answer to a questionnaire item as to the living subject is inputted on the degree-of-health outputting device and information on it is transmitted to the information providing device, a remedy suitable for the determined degree of health of the living subject and the answer to the questionnaire item, and the communication unit may be configured to transmit information on the degree of health and the remedy determined by the degree-of-health determination unit to the degree-of-health outputting device.

The degree-of-health outputting device can receive information on the remedy determined by the information providing device and output it on the output unit. This helps reduce the burden of processing for determining the remedy on the degree-of-health outputting device.

As described above, according to another embodiment, a degree-of-health outputting device includes: a display unit configured to display together a shot image obtained by shooting a part whose condition changes according to the degree of health in a living subject, a plurality of comparison images with different degrees of health obtained based on a reference image (created from a reference image by image processing) obtained by shooting the part before the time of acquisition of the shot image with respect to the same living subject, and information as to the degree of health of the living subject corresponding to each of the plurality of comparison images.

Thus, the shot image and the comparison targets (the plurality of comparison images) are ones as to the same living subject, and accordingly the outputted degree of health excludes the influence of an individual difference from other living subjects. This enables the user to grasp accurately the degree of health (level of disease) of the living subject. In addition, the plurality of comparison images are obtained based on the reference image (created by image processing), and even a condition that falls outside the range of variation of the examinee's health condition in the past can be presented as a comparison image; thus, even if the examinee's health condition at the time of diagnosis falls outside the range of variation of physical condition in the past, it is possible to grasp accurately the examinee's degree of health. Moreover, information on the degree of health of the living subject that corresponds to the comparison image is displayed, and thus it is possible to see the examinee's degree of health at a glance.

As described above, according to another embodiment, a degree-of-health outputting system includes: a degree-of-health outputting device as described above; and an information providing device. Here, the information providing device includes: a comparison image creation unit configured to create the plurality of comparison images from the reference image by image processing; a storage unit configured to store a table defining the correspondence between each of the plurality of comparison images and the information as to the degree of health; and a communication unit configured to output the data of the plurality of comparison images and the information as to the degree of health corresponding to each of the plurality of comparison images to the degree-of-health outputting device.

In the above system, the degree-of-health outputting device can receive the data of the plurality of comparison images created in the information providing device and information as to the degree of health, display them on the display unit, and thereby immediately notify the user of the degree of health.

**Industrial Applicability**

The present invention find applications in systems for assisting diagnosis of the health condition of living subjects.

**List of Reference Signs**

| | |
|---|---|
| 1 | degree-of-health outputting device |
| 1a | organ image shooting device (degree-of-health outputting device) |
| 1b | organ image shooting device (degree-of-health outputting device) |
| 2 | illuminating unit |
| 3 | imaging unit |
| 4 | display unit (output unit) |
| 5 | operation unit (input unit) |
| 6 | detection unit |
| 7 | communication unit |
| 8 | audio output unit (output unit) |
| 18 | image processing unit (correction unit) |
| 19 | determination unit |
| 50 | reference image outputting device |
| 60 | information providing device |
| 61 | comparison image creation unit |
| 62 | image correction unit |
| 64 | degree-of-health determination unit |
| 65 | communication unit |
| 100 | degree-of-health outputting system |
| NW | communication network |

**Claims**

1. A degree-of-health outputting device, comprising:

   a display unit configured to display together

      a shot image obtained by shooting a part whose condition changes according to a degree of health in a living subject and
      a plurality of comparison images with different degrees of health obtained based on a reference image obtained by shooting the part before a time of acquisition of the shot image with respect to the same living subject;

   an operation unit configured to be operated to select one of the plurality of comparison images displayed on the display unit; and
   an output unit configured to output information as to the degree of health of the living subject based on the comparison image selected on the operation unit.

**2.** The degree-of-health outputting device of claim 1, wherein
the reference image is an image shot in healthy condition with respect to the same living subject.

**3.** The degree-of-health outputting device of claim 1 or 2, wherein
the plurality of comparison images are images obtained by changing the reference image over a plurality of grades with different degrees of health with respect to at least one diagnosis item that serves as an index in determining the degree of health of the living subject.

**4.** The degree-of-health outputting device of claim 3, wherein
the part is a tongue of the living subject, and
the diagnosis item includes at least one of tongue color, tongue shape, tongue coating color, and tongue coating thickness.

**5.** The degree-of-health outputting device of claim 3 or 4, wherein
the at least one diagnosis item comprises a plurality of diagnosis items,
the display unit is configured to display, for each of the plurality of diagnosis items, the plurality of comparison images obtained with respect to the each of the plurality of diagnosis items, and
the output unit is configured to output, for each of the plurality of diagnosis items, the information as to the degree of health of the living subject based on the comparison image selected for the each of the plurality of diagnosis items on the operation unit.

**6.** The degree-of-health outputting device of any one of claims 1 to 5, wherein
the display unit is configured to further display a questionnaire item as to the living subject,
the degree-of-health outputting device further comprises an input unit configured to accept input of an answer to the questionnaire item displayed on the display unit, and
the output unit is configured to output, along with the information as to the degree of health, a remedy suitable for the outputted degree of health of the living subject and the answer inputted on the input unit.

**7.** The degree-of-health outputting device of any one of claims 1 to 6, further comprising:

an image processing unit configured to create the plurality of comparison images from the reference image by image processing.

**8.** The degree-of-health outputting device of any one of claims 1 to 7, further comprising:

a determination unit configured to determine the degree of health of the living subject based on the comparison image selected on the operation unit, wherein
the output unit is configured to output information as to the degree of health determined by the determination unit.

**9.** A program for making a computer execute:

a process of displaying on a display unit

a shot image obtained by shooting a part whose condition changes according to a degree of health in a living subject and
a plurality of comparison images with different degrees of health obtained based on a reference image obtained by shooting the part before a time of acquisition of the shot image with respect to the same living subject; and

a process of, when one of the plurality of comparison images displayed on the display unit is selected on an operation unit, outputting, from an output unit, information as to the degree of health of the living subject based on the selected comparison image.

**10.** A degree-of-health outputting system, comprising:

the degree-of-health outputting device of any one of claims 1 to 6; and
an information providing device, wherein
the information providing device includes:

a comparison image creation unit configured to create the plurality of comparison images from the reference image by image processing; and
a communication unit configured to output data of the plurality of comparison images to the degree-of-health outputting device.

11. The degree-of-health outputting system of claim 10, further comprising:

a reference image outputting device configured to transmit data of the reference image and information as to a color temperature of an ambient light at a time of acquisition of the reference image to the information providing device, wherein
the degree-of-health outputting device includes:

an imaging unit configured to acquire the shot image by shooting the part of the living subject;
an illumination unit configured to illuminate the part of the living subject at a time of shooting by the imaging unit;
a detection unit configured to detect a color temperature of light around the part illuminated by the illumination unit; and
a communication unit configured to transmit image data of the shot image acquired by the imaging unit and information on the color temperature detected by the detection unit to the information providing device,

the information providing device further includes:

an image correction unit configured to correct at least either the shot image or the plurality of comparison images such that chromaticity of an entire image of each of the comparison images is closer, in relative terms, to chromaticity of an entire image of the shot image when the color temperature at the time of acquisition of the shot image transmitted from the degree-of-health outputting device differs from the color temperature at the time of acquisition of the reference image transmitted from the reference image outputting device, and

the communication unit in the information providing device is configured to transmit data of an image or images corrected by the image correction unit to the degree-of-health outputting device.

12. The degree-of-health outputting system of claim 11, wherein
the image correction unit in the information providing device is configured to correct at least either the shot image or the plurality of comparison images such that an average value of image data of each color in each of the comparison images is closer, in relative terms, to an average value of image data of each color in the shot image.

13. The degree-of-health outputting system of claim 10, further comprising:

a reference image outputting device configured to transmit data of the reference image and information as to a color temperature of an ambient light at a time of acquisition of the reference image to the information providing device, wherein
the communication unit in the information providing device is configured to transmit the information on the color temperature at the time of acquisition of the reference image transmitted from the reference image outputting device along with the data of the plurality of comparison images to the degree-of-health outputting device, and
the degree-of-health outputting device includes:

an imaging unit configured to acquire the shot image by shooting the part of the living subject;
an illumination unit configured to illuminate the part of the living subject at a time of shooting by the imaging unit;
a detection unit configured to detect a color temperature of light around the part illuminated by the illumination unit; and
a correction unit configured to correct at least either the shot image or the plurality of comparison images such that chromaticity of an entire image of each of the comparison images is closer, in relative terms, to chromaticity of an entire image of the shot image when the color temperature at the time of acquisition of the shot image detected by the detection unit differs from the color temperature at the time of acquisition of the reference image transmitted from the information providing device.

**14.** The degree-of-health outputting system of claim 13, wherein
the correction unit in the degree-of-health outputting device is configured to correct at least either the shot image or the plurality of comparison images such that an average value of image data of each color in each of the comparison images is closer, in relative terms, to an average value of image data of each color in the shot image.

**15.** The degree-of-health outputting device of any one of claims 10 to 14, wherein
the information providing device further includes:

a degree-of-health determination unit configured to determine the degree of health of the living subject based on the comparison image selected on the operation unit in the degree-of-health outputting device, wherein

the communication unit is configured to transmit information on the degree of health determined by the degree-of-health determination unit to the degree-of-health outputting device.

**16.** The degree-of-health outputting device of claim 15, wherein
the degree-of-health determination unit is configured to determine, when an answer to a questionnaire item as to the living subject is inputted on the degree-of-health outputting device and information thereon is transmitted to the information providing device, a remedy suitable for the determined degree of health of the living subject and the answer to the questionnaire item, and
the communication unit is configured to transmit information on the degree of health and the remedy determined by the degree-of-health determination unit to the degree-of-health outputting device.

**17.** A degree-of-health outputting device, comprising:

a display unit configured to display together

a shot image obtained by shooting a part whose condition changes according to a degree of health in a living subject,
a plurality of comparison images with different degrees of health obtained based on a reference image obtained by shooting the part before a time of acquisition of the shot image with respect to the same living subject, and
information as to the degree of health of the living subject corresponding to each of the plurality of comparison images.

**18.** A degree-of-health outputting system, comprising:

the degree-of-health outputting device of claim 17; and
an information providing device, wherein
the information providing device includes:

a comparison image creation unit configured to create the plurality of comparison images from the reference image by image processing;
a storage unit configured to store a table defining correspondence between each of the plurality of comparison images and the information as to the degree of health; and
a communication unit configured to output data of the plurality of comparison images and the information as to the degree of health corresponding to each of the plurality of comparison images to the degree-of-health outputting device.

EP 3 213 672 A1

# FIG.1

REFERENCE IMAGE
OUTPUTTING DEVICE
50

DEGREE-OF-HEALTH
OUTPUTTING DEVICE
1

INFORMATION
PROVIDING DEVICE
60

NW

100

# FIG.2

1(1a)

6

2

21

5(5a) OK CANCEL 5(5b)

22

41

4

3

25

# FIG.3

1(1a)

```
                    ┌──────────────┐        ┌──────────────┐
                ┌──▶│ ILLUMINATION │───────▶│ ILLUMINATION │
                │   │ CONTROL UNIT │        │     UNIT     │
                │   └──────────────┘        └──────────────┘
                │              9                        2
                │   ┌──────────────┐        ┌──────────────┐
                ├──▶│   IMAGING    │───────▶│ IMAGING UNIT │
                │   │ CONTROL UNIT │        │              │
                │   └──────────────┘        └──────────────┘
                │             10                        3
                │   ┌──────────────┐        ┌──────────────┐
                ├──▶│   DISPLAY    │───────▶│ DISPLAY UNIT │
                │   │ CONTROL UNIT │        │              │
                │   └──────────────┘        └──────────────┘
                │             11                        4
                │   ┌──────────────┐        ┌──────────────┐
                ├──▶│  OPERATION   │───────▶│  OPERATION   │
                │   │ CONTROL UNIT │        │     UNIT     │
                │   └──────────────┘        └──────────────┘
                │             12                        5
┌───────────┐  │   ┌──────────────┐        ┌──────────────┐
│  OVERALL  │◀─┤──▶│  DETECTION   │───────▶│  DETECTION   │
│  CONTROL  │  │   │ CONTROL UNIT │        │     UNIT     │
│   UNIT    │  │   └──────────────┘        └──────────────┘
└───────────┘  │             13                        6
      20       │   ┌──────────────┐
               ├──▶│ CALCULATION  │
               │   │     UNIT     │
               │   └──────────────┘
               │             14
               │   ┌──────────────┐
               ├──▶│ STORAGE UNIT │
               │   └──────────────┘
               │             15
               │   ┌──────────────┐        ┌──────────────┐
               ├──▶│COMMUNICATION │───────▶│COMMUNICATION │
               │   │ CONTROL UNIT │        │     UNIT     │
               │   └──────────────┘        └──────────────┘
               │             16                        7
               │   ┌──────────────┐        ┌──────────────┐
               └──▶│ SOUND OUTPUT │───────▶│ AUDIO OUTPUT │
                   │ CONTROL UNIT │        │     UNIT     │
                   └──────────────┘        └──────────────┘
                             17                        8
```

FIG.4

# FIG.5

SHOT IMAGE

4

EDGE EXTRACTION FILTER

| | -1 | |
|---|---|---|
| -1 | 4 | -1 |
| | -1 | |

EXTRACTED CONTOUR
LINE

# FIG.6

50

| DISPLAY UNIT | 51 |
| OPERATION UNIT | 52 |
| REFERENCE IMAGE IMAGING UNIT | 53 |
| IMAGE PROCESSING UNIT | 54 |
| STORAGE UNIT | 55 |
| COMMUNICATION UNIT | 56 |

CONTROL UNIT

57

# FIG.7

60

```
                    ┌──────────────────────┐
                ┌──→│  COMPARISON IMAGE     │
                │   │  CREATION UNIT        │
                │   └──────────────────────┘
                │                        61
                │   ┌──────────────────────┐
                ├──→│  IMAGE CORRECTION     │
                │   │       UNIT            │
                │   └──────────────────────┘
  ┌──────────┐  │                        62
  │          │←─┤   ┌──────────────────────┐
  │ CONTROL  │  ├──→│    STORAGE UNIT       │
  │  UNIT    │  │   └──────────────────────┘
  │          │  │                        63
  └──────────┘  │   ┌──────────────────────┐
       66       ├──→│  DEGREE-OF-HEALTH     │
                │   │  DETERMINATION        │
                │   │       UNIT            │
                │   └──────────────────────┘
                │                        64
                │   ┌──────────────────────┐
                └──→│  COMMUNICATION        │
                    │       UNIT            │
                    └──────────────────────┘
                                        65
```

EP 3 213 672 A1

# FIG.8

| DIAGNOSIS ITEMS ADOPTED BY KAMPO DOCTORS, AND NUMBERS OF GRADES | | | | |
|---|---|---|---|---|
| TARGET | ITEM | DESCRIPTION | GRADES | STATE VARIATION |
| TONGUE | COLOR | BASE COLOR | 4 | REDDISH-PURPLE /PALE WHITE/PALE PINK/ DEEP PINK |
| | THICKNESS | TONGUE THICKNESS | 3 | THIN/ADEQUATE/THICK |
| | MOISTNESS | GLOSS ON SURFACE | 3 | NO/ADEQUATE/HIGH |
| | SHAPE (TOOTH MARKS) | IRREGULARITIES IN SIDE | 4 | NONE/FEW/MEDIUM/MANY |
| | FISSURES (CRACKS) | MIDDLE DARK LINE | 4 | NONE/FEW/MEDIUM/MANY |
| TONGUE COATING | COLOR | COATING COLOR | 4 | PATE WHITE/WHITE/ YELLOW/BROWN |
| | SMOOTHNESS | PAPILLA TISSUE SEPARATION | 2 | LOW/HIGH |
| | THICKNESS | COATING THICKNESS | 3 | THIN/ADEQUATE/THICK |
| | PRESENCE/ ABSENCE | COATING PRESENCE/ ABSENCE | 2 | ABSENT/PRESENT |
| | DISTRIBUTION | COATING DISTRIBUTION | 2 | EVEN/UNEVEN |

◯ : INDICATES NORMAL STATE

31

# FIG.9

COMPARISON IMAGES AS TO TONGUE COLOR

| COMPARISON IMAGE 1-1 | COMPARISON IMAGE 1-2 | COMPARISON IMAGE 1-3 | COMPARISON IMAGE 1-4 |

REGION A1 — REDDISH PURPLE — $X1=(R1,G1)$ — LEVEL 1 ABNORMAL

REGION A1 — PALE WHITE — $X2=(R2,G2)$ — LEVEL 2 ABNORMAL

REGION A1 — PALE PINK — $X3=(R3,G3)$ — LEVEL 3 NORMAL

REGION A1 — DEEP PINK — $X4=(R4,G4)$ — LEVEL 4 ABNORMAL

EP 3 213 672 A1

# FIG.10

RG CHROMATICITY
COORDINATE

$G=-0.5R+C$

# FIG.11

# FIG.12

EP 3 213 672 A1

COMPARISON IMAGES AS TO TONGUE SHAPE
(TOOTH MARKS)

| COMPARISON IMAGE 2-1 | COMPARISON IMAGE 2-2 | COMPARISON IMAGE 2-3 | COMPARISON IMAGE 2-4 |
|---|---|---|---|
| LEVEL 1 NORMAL | LEVEL 2 ABNORMAL | LEVEL 3 ABNORMAL | LEVEL 4 ABNORMAL |

# FIG.13

COMPARISON IMAGES AS TO TONGUE COATING COLOR

| COMPARISON IMAGE 3-1 | COMPARISON IMAGE 3-2 | COMPARISON IMAGE 3-3 | COMPARISON IMAGE 3-4 |
|---|---|---|---|

| PALE WHITE REGION A3 | WHITE REGION A3 | YELLOW REGION A3 | BROWN REGION A3 |
|---|---|---|---|
| $Y1=(R1,G1)$ | $Y2=(R2,G2)$ | $Y3=(R3,G3)$ | $Y4=(R4,G4)$ |
| LEVEL 1 NORMAL | LEVEL 2 ABNORMAL | LEVEL 3 ABNORMAL | LEVEL 4 ABNORMAL |

EP 3 213 672 A1

FIG.14

RG CHROMATICITY
COORDINATE

# FIG.15

COMPARISON IMAGES AS TO TONGUE COATING
THICKNESS

| COMPARISON IMAGE 4-1 | COMPARISON IMAGE 4-2 | COMPARISON IMAGE 4-3 |
|---|---|---|
| THIN REGION A2 | ADEQUATE REGION A2 | THICK REGION A2 |
| LEVEL 1 ABNORMAL | LEVEL 2 NORMAL | LEVEL 3 ABNORMAL |

EP 3 213 672 A1

# FIG.16

REFERENCE IMAGE 1

ILLUMINATION
LIGHT SOURCE
FLASH

REFERENCE IMAGE 2

ILLUMINATION
LIGHT SOURCE
FLUORESCENT LAMP

REFERENCE IMAGE 3

ILLUMINATION
LIGHT SOURCE
INCANDESCENT LAMP

EP 3 213 672 A1

# FIG.17

COMPARISON IMAGES AS TO TONGUE COLOR

COMPARISON IMAGE
1-1

COMPARISON IMAGE
1-2

COMPARISON IMAGE
1-3

COMPARISON IMAGE
1-4

↓CORRECTION

↓CORRECTION

↓CORRECTION

↓CORRECTION

EP 3 213 672 A1

# FIG.18

PROCESSING ON REFERENCE
IMAGE OUTPUTTING DEVICE

PROCESSING ON INFORMATION
PROVIDING DEVICE

START

TURN ON ILLUMINATION
S1

DETECT COLOR
TEMPERATURE
S2

SHOOT
S3

CUT OUT IMAGE
S4

STORE IMAGE DATA & COLOR
TEMPERATURE
S5

TRANSMIT INFORMATION TO
INFORMATION OUTPUTTING
DEVICE
S6

CREATE COMPARISON
IMAGES
S7

STORE DATA OF
COMPARISON IMAGES
S8

END

EP 3 213 672 A1

# FIG.19

PROCESSING ON DEGREE-OF-
HEALTH OUTPUTTING DEVICE

PROCESSING ON INFORMATION
PROVIDING DEVICE

START

TURN ON ILLUMINATION
S11

DETECT COLOR
TEMPERATURE
S12

SHOOT
S13

TRANSMIT INFORMATION
TO INFORMATION
PROVIDING DEVICE
S14

COLOR
TEMPERATURES
DIFFER? — N
S15
Y

CORRECT IMAGE(S)
S16

TRANSMIT IMAGE DATA
TO DEGREE-OF-HEALTH
OUTPUTTING DEVICE
S17

DISPLAY SHOT IMAGE &
COMPARISON IMAGES ON
DEGREE-OF-HEALTH
OUTPUTTING DEVICE
S18

ACCEPT SELECTION OF
COMPARISON IMAGE
S19

TRANSMIT INFORMATION
ON SELECTED COMPARISON
IMAGE TO INFORMATION
PROVIDING DEVICE
S20

DETERMINE OBSERVATION
S21

DETERMINE PHYSICAL
CONDITION
S22

STORE INFORMATION ON
DEGREE OF HEALTH
S23

OUTPUT INFORMATION ON
DEGREE OF HEALTH
S25

TRANSMIT INFORMATION
ON DEGREE OF HEALTH
TO DEGREE-OF-HEALTH
OUTPUTTING DEVICE
S24

END

41

# FIG.20

# FIG.21

COMPARISON
IMAGE 2-1

COMPARISON
IMAGE 2-2

COMPARISON
IMAGE 2-3

COMPARISON
IMAGE 2-4

41

4

SHOT IMAGE

PALE
WHITE

# FIG.22

PROCESSING ON DEGREE-OF-
HEALTH OUTPUTTING DEVICE

PROCESSING ON INFORMATION
PROVIDING DEVICE

( START )

TURN ON ILLUMINATION
S11

DETECT COLOR TEMPERATURE
S12

SHOOT
S13

TRANSMIT INFORMATION TO
INFORMATION PROVIDING
DEVICE
S14

COLOR
TEMPERATURES
DIFFER?          N
                 S15
Y

CORRECT IMAGE(S)
S16

DISPLAY SHOT IMAGE &
COMPARISON IMAGES ON
DEGREE-OF-HEALTH
OUTPUTTING DEVICE
S18

TRANSMIT IMAGE DATA
TO DEGREE-OF-HEALTH
OUTPUTTING DEVICE
S17

ACCEPT SELECTION OF
COMPARISON IMAGE
S19

DISPLAY
QUESTIONNAIRE ITEMS
S19-1

ACCEPT ANSWERS
S19-2

DETERMINE
OBSERVATION
S21

DETERMINE PHYSICAL
CONDITION & REMEDY
S22'

TRANSMIT INFORMATION ON
SELECTED COMPARISON IMAGE
& ANSWERS TO QUESTIONNAIRE
ITEMS TO INFORMATION
PROVIDING DEVICE
S20'

STORE INFORMATION
S23'

OUTPUT INFORMATION ON
DEGREE OF HEALTH & REMEDY
S25'

TRANSMIT INFORMATION
ON DEGREE OF HEALTH &
REMEDY TO DEGREE-OF-
HEALTH OUTPUTTING
DEVICE
S24'

( END )

# FIG.23

QUESTIONNAIRE SHEET

[QUESTIONNAIRE ITEMS]

1. BODY TEMPERATURE _____ °C

2. HAVE HEADACHE?    [ YES ]    [ NO ]

3. FEEL SICK?    [ YES ]    [ NO ]

4. FEEL DIZZY?    [ YES ]    [ NO ]

5. FEEL LANGUOR?    [ YES ]    [ NO ]

6. SINCE WHEN FEEL UNWELL?

SINCE ABOUT (YEAR) (MONTH) (DAY)

[ NORMAL ]

⋮

~ 4

~ 41

| NUM | 1 | 2 | 3 | × |
| A | 4 | 5 | 6 | ENTER |
| KANA | 7 | 8 | 9 | |
| SYMBOLS | – | 0 | SPACE | |

← 5

# FIG.24

COMPARISON IMAGE 1-1
LEVEL 1, NORMAL

PALE WHITE

COMPARISON IMAGE 1-2
LEVEL 2,
SLIGHTLY ABNORMAL

WHITE

COMPARISON IMAGE 1-3
LEVEL 3, ABNORMAL

YELLOW

COMPARISON IMAGE 1-4
LEVEL 4,
SERIOUSLY ABNORMAL

BROWN

~41

~4

SHOT IMAGE

PALE WHITE

# FIG.25

100

REFERENCE IMAGE
OUTPUTTING DEVICE

50

DEGREE-OF-HEALTH
OUTPUTTING DEVICE

1

NW

# FIG.26

1(1b)

| | | |
|---|---|---|
| | ILLUMINATION CONTROL UNIT ⌐9 | ILLUMINATION UNIT ⌐2 |
| | IMAGING CONTROL UNIT ⌐10 | IMAGING UNIT ⌐3 |
| | DISPLAY CONTROL UNIT ⌐11 | DISPLAY UNIT ⌐4 |
| OVERALL CONTROL UNIT | OPERATION CONTROL UNIT ⌐12 | OPERATION UNIT ⌐5 |
| 20 | DETECTION CONTROL UNIT ⌐13 | DETECTION UNIT ⌐6 |
| | CALCULATION UNIT ⌐14 | |
| | STORAGE UNIT ⌐15 | |
| | COMMUNICATION CONTROL UNIT ⌐16 | COMMUNICATION UNIT ⌐7 |
| | SOUND OUTPUT CONTROL UNIT ⌐17 | AUDIO OUTPUT UNIT ⌐8 |
| | IMAGE PROCESSING UNIT ⌐18 | |
| | DETERMINATION UNIT ⌐19 | |

# FIG.27

PROCESSING ON REFERENCE
IMAGE OUTPUTTING DEVICE

PROCESSING ON DEGREE-OF-
HEALTH OUTPUTTING DEVICE

START

TURN ON
ILLUMINATION
S31

DETECT COLOR
TEMPERATURE
S32

SHOOT
S33

CUT OUT IMAGE
S34

STORE IMAGE DATA &
COLOR TEMPERATURE
S35

TRANSMIT
INFORMATION TO
DEGREE-OF-HEALTH
OUTPUTTING DEVICE
S36

CREATE COMPARISON
IMAGES
S37

STORE DATA OF
COMPARISON IMAGES
S38

END

# FIG.28

PROCESSING ON DEGREE-OF-HEALTH
OUTPUTTING DEVICE

```
                    ┌─────────────┐
                    │    START    │
                    └─────────────┘
                           │
                           ▼
        ┌──────────────────────────────────┐
        │      TURN ON ILLUMINATION         │
        └──────────────────────────────────┘  S41
                           │
                           ▼
        ┌──────────────────────────────────┐
        │     DETECT COLOR TEMPERATURE      │
        └──────────────────────────────────┘  S42
                           │
                           ▼
        ┌──────────────────────────────────┐
        │              SHOOT                │
        └──────────────────────────────────┘  S43
                           │
                           ▼
                    ╱╲
                   ╱  COLOR  ╲          N
                  ╱ TEMPERATURES ╲──────────┐
                   ╲  DIFFER? ╱             │
                    ╲╱  S44                 │
                     │ Y                    │
                     ▼                      │
        ┌──────────────────────────────────┐│
        │        CORRECT IMAGE(S)           ││
        └──────────────────────────────────┘│ S45
                           │◄───────────────┘
                           ▼
```

```
┌──────────────────────────┐       ┌──────────────────────────────┐
│  DISPLAY SHOT IMAGE &     │       │    DETERMINE OBSERVATION       │
│  COMPARISON IMAGES        │  S46  └──────────────────────────────┘ S50
└──────────────────────────┘                       │
            │                                       ▼
            ▼                       ┌──────────────────────────────┐
┌──────────────────────────┐       │ DETERMINE PHYSICAL CONDITION   │
│  ACCEPT SELECTION OF      │       │        & REMEDY                │
│  COMPARISON IMAGE         │  S47  └──────────────────────────────┘ S51
└──────────────────────────┘                       │
            │                                       ▼
            ▼                       ┌──────────────────────────────┐
┌──────────────────────────┐       │       STORE INFORMATION        │
│ DISPLAY QUESTIONNAIRE ITEMS│ S48  └──────────────────────────────┘ S52
└──────────────────────────┘                       │
            │                                       ▼
            ▼                       ┌──────────────────────────────┐
┌──────────────────────────┐       │  OUTPUT DEGREE OF HEALTH &     │
│      ACCEPT ANSWERS       │       │        REMEDY                  │
└──────────────────────────┘  S49  └──────────────────────────────┘ S53
            │                                       │
            └──────────────────────►                ▼
                                              ┌─────────────┐
                                              │     END     │
                                              └─────────────┘
```

# FIG.29

72

71

# FIG.30

COMPARISON IMAGE
5-1

COMPARISON IMAGE
5-2

COMPARISON IMAGE
5-3

## INTERNATIONAL SEARCH REPORT

| International application No. |
| --- |
| PCT/JP2015/078711 |

A. CLASSIFICATION OF SUBJECT MATTER
**A61B5/00**(2006.01)i, **A61B5/107**(2006.01)i

According to International Patent Classification (IPC) or to both national classification and IPC

B. FIELDS SEARCHED

Minimum documentation searched (classification system followed by classification symbols)
A61B5/00-5/01

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2005-137756 A (Konica Minolta Holdings, Inc.), 02 June 2005 (02.06.2005), paragraphs [0007] to [0009] (Family: none) | 1–18 |
| A | JP 2006-149679 A (Konica Minolta Holdings, Inc.), 15 June 2006 (15.06.2006), paragraphs [0009], [0011] (Family: none) | 1–18 |
| A | JP 2004-344583 A (Minolta Co., Ltd.), 09 December 2004 (09.12.2004), paragraphs [0007], [0071] (Family: none) | 1–18 |

☐ Further documents are listed in the continuation of Box C.　　☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 December 2015 (10.12.15) | 22 December 2015 (22.12.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013017660 A
- JP 5150440 B
- JP 2763989 B
- JP 2007279942 A